# EUROPÄISCHE PATENTSCHRIFT

(11) **EP 1 289 920 B1**
(45) Veröffentlichungstag und Bekanntmachung des Hinweises auf die Patenterteilung: **15.09.2004**
(21) Anmeldenummer: 01960291.1
(22) Anmeldetag: 08.06.2001
(51) Int. Cl.: C07C 45/33, C07C 45/35, C07C 47/22, C07C 51/215, C07C 51/25, C07C 57/04, C07C 5/333, C07C 11/06

(54) **VERFAHREN ZUR HERSTELLUNG VON ACROLEIN ODER ACRYLSÄURE ODER DEREN GEMISCH AUS PROPAN**
METHOD FOR THE PRODUCTION OF ACROLEIN OR ACRYLIC ACID OR THE MIXTURE THEREOF FROM PROPANE
PROCEDE DE PRODUCTION D'ACROLEINE OU D'ACIDE ACRYLIQUE OU DE LEUR MELANGE A PARTIR DE PROPANE

(30) Priorität: 14.06.2000 DE 10028582
(43) Veröffentlichungstag der Anmeldung: 12.03.2003
(73) Patentinhaber: BASF AKTIENGESELLSCHAFT, 67056 Ludwigshafen (DE)
(72) Erfinder: MACHHAMMER, Otto, D-68163 Mannheim (DE); SCHINDLER, Goetz-Peter, D-68219 Mannheim (DE); TENTEN, Andreas, D-67487 Maikammer (DE); HARTH, Klaus, D-67317 Altleiningen (DE); ZEHNER, Peter, D-67071 Ludwigshafen (DE); MÜLLER-ENGEL, Klaus, Joachim, D-76297 Stutensee (DE); ROSOWSKI, Frank, D-68165 Mannheim (DE); BORGMEIER, Frieder, D-68163 Mannheim (DE)
(86) Internationale Anmeldenummer: PCT/EP2001/006528
(87) Internationale Veröffentlichungsnummer: WO 2001/096270

(56) Entgegenhaltungen:
- EP-A- 0 731 077
- EP-A- 0 731 082
- DE-A- 19 622 331
- GB-A- 2 118 939
- US-A- 5 198 578
- DATABASE WPI Section Ch, Derwent Publications Ltd., London, GB; Class A00, AN 1968-04476Q XP002190603 & SU 193 484 A (LA IVANOVA)

## Beschreibung

Vorliegende Erfindung betrifft ein Verfahren zur Herstellung von Acrolein oder Acrylsäure oder deren Gemisch aus Propan, bei dem man
A) in einer ersten Stufe A das Propan einer partiellen heterogen katalysierten Dehydrierung in der Gasphase unter Bildung eines Produktgasgemisches A, das molekularen Wasserstoff, Propylen und nicht umgesetztes Propan enthält, unterwirft,
B) aus dem molekularen Wasserstoff, Propylen und nicht umgesetztes Propan enthaltenden Produktgasgemisch A der Stufe A von den darin enthaltenen, von Propan und Propylen verschiedenen, Bestandteilen wenigstens eine Teilmenge des molekularen Wasserstoff abtrennt und es dann als Produktgasgemisch A' in einer zweiten Stufe B zur Beschickung wenigstens eines Oxidationsreaktors verwendet und in dem wenigstens einen Oxidationsreaktor das Propylen einer selektiven heterogen katalysierten Gasphasen-Partialoxidation mit molekularem Sauerstoff zu einem Produktgasgemisch B, das Acrolein oder Acrylsäure oder deren Gemisch als zielprodukt enthält, unterwirft, und
C) aus dem im Rahmen der partiellen Oxidation des Propylens in der Stufe B anfallenden Produktgasgemisch B in einer dritten Stufe C Zielprodukt abtrennt und wenigstens im Produktgasgemisch der Stufe B enthaltenes nicht umgesetztes Propan in die Dehydrierungsstufe A zurückführt.

Acrylsäure ist eine bedeutende Grundchemikalie, die unter anderem als Monomeres zur Herstellung von Polymerisaten Verwendung findet, die beispielsweise in disperser Verteilung in wäßrigem Medium befindlich als Bindemittel angewendet werden. Acrolein ist ein bedeutendes Zwischenprodukt, beispielsweise für die Herstellung von Glutardialdehyd, Methionin, Folsäure und Acrylsäure.

-Aus der EP-A 117 146, der DE-A 3 313 573 und der US-A 3 161 670 ist ein Verfahren zur Umsetzung von Propan zu Acrolein und/oder Acrylsäure bekannt (nachfolgend wird sich repräsentativ nur noch auf die EP-A 117 146 bezogen).

Dabei wird in einer ersten Verfahrensstufe das Propan einer heterogen katalysierten partiellen Dehydrierung in der Gasphase zu Propylen unterzogen. Das dabei gebildete Propylen wird anschließend in einer zweiten Verfahrensstufe einer heterogen katalysierten Gasphasen-Partialoxidation zu Acrolein und/oder Acrylsäure unterworfen. Kennzeichnendes Merkmal der EP-A 117 146 ist die Lehre, daß sich die neben Propylen im Produktgasgemisch der Propandehydrierung befindlichen Hauptbestandteile, wie z.B. molekularer Wasserstoff, bezüglich der nachfolgenden heterogen katalysierten Gasphasen-Partialoxidation des Propylens im wesentlichen inert verhalten, so daß man das Produktgasgemisch der Propandehydrierung.gemäß der EP-A 117 146 ohne wesentliche Nachteile in seiner Gesamtheit in die nachfolgende Propylenoxidationsstufe überführen und von den sich dabei inert verhaltenden Bestandteilen anschließend wenigstens das nicht umgesetzte Propan in die Propandehydrierungsstufe rückführen kann.

Die DE-A 19 508 558 weist die Lehre der EP-A 117 146 insofern als nachteilig aus, als sie lehrt, daß die Mitverwendung von von Propan verschiedenen inerten Verdünnungsgasen in der zweiten Verfahrensstufe der EP-A 117 146 unvorteilhaft ist. Kennzeichnendes Merkmal der DE-A 19 508 558 ist es daher, aus dem Produktgasgemisch der ersten Verfahrensstufe der EP-A 117 146 vor seiner Weiterverwendung in der zweiten Verfahrensstufe wenigstens den molekularen Wasserstoff und den Wasserdampf abzutrennen und als Sauerstoffquelle für die zweite Verfahrensstufe reinen Sauerstoff zu verwenden.

Als Ergebnis intensiver Forschungstätigkeit wurde jedoch gefunden, daß die in der DE-A 19 508 558 empfohlene Verfahrensweise in der zweiten Verfahrensstufe eine erhöhte Nebenproduktbildung von Propionaldehyd und/oder Propionsäure bedingt. Letzteres ist insofern von Nachteil (vgl. z.B. Japanese Laid Open Patent Application No. H11-35519), als die gesättigten partiellen C₃-Oxidationsnebenprodukte von den α,β-ethylenisch ungesättigten partiellen C₃-Oxidationszielprodukten einerseits infolge ihrer chemischen Ähnlichkeit nur sehr schwierig abgetrennt werden können und andererseits aufgrund ihres penetranten Geruchs bereits in geringsten Anteilmengen bei der Vermarktung der partiellen C₃-Oxidationszielprodukte empfindlichst stören.

Die Aufgabe der vorliegenden Erfindung bestand daher darin, eine gegenüber den Verfahrensweisen der EP-A 117 146 sowie der DE-A 19 508 558 verbesserte Verfahrensweise zur Verfügung zu stellen.

Als solche vermögen z.B. die Verfahrensweise der DE-A 19 837 517, der DE-A 19 837 519 sowie der DE-A 19 837 520, bei denen der Verfahrensschritt der heterogen katalysierten partiellen Dehydrierung durch eine homogene und/oder heterogen katalysierte partielle Oxidehydrierung ersetzt ist, nicht zu befriedigen (obgleich das dabei resultierende Dehydriergemisch nicht wasserstoffhaltig ist), bedürfen sie doch bereits im Rahmen der Dehydrierverfahrensstufe der Mitverwendung erheblicher Mengen an molekularem Sauerstoff.

Demgemäß wurde ein Verfahren zur Herstellung von Acrolein oder Acrylsäure oder deren Gemisch aus Propan, bei dem man
A) in einer ersten Stufe A das Propan einer partiellen heterogen katalysierten Dehydrierung in der Gasphase unter Bildung eines Produktgasgemisches A, das molekularen Wasserstoff, Propylen und nicht umgesetztes Propan enthält, unterwirft,
B) aus dem molekularen Wasserstoff, Propylen und nicht umgesetztes Propan enthaltenden Produktgasgemisch A der Stufe A von den darin enthaltenen, von Propan und Propylen verschiedenen Bestandteilen wenigstens eine Teilmenge des molekularen Wasserstoff abtrennt und es dann als Produktgasgemisch A' in einer zweiten Stufe B zur Beschickung wenigstens eines Oxidationsreaktors verwendet und in dem wenigstens einen Oxidationsreaktor das Propylen einer selektiven heterogen katalysierten Gasphasen-Partialoxidation mit molekularem Sauerstoff zu einem Produktgasgemisch B, das Acrolein oder Acrylsäure oder deren Gemisch als Zielprodukt enthält, unterwirft, und
C) aus dem im Rahmen der partiellen Oxidation des Propylens in der Stufe B anfallenden Produktgasgemisch B in einer dritten Stufe C Zielprodukt abtrennt und wenigstens im Produktgasgemisch der Stufe B enthaltenes nicht umgesetztes Propan in die Dehydrierungsstufe A zurückführt, gefunden,
das dadurch gekennzeichnet ist, daß im Rahmen der partiellen Oxidation des Propylens in der Stufe B molekularer Stickstoff als Verdünnungsgas mitverwendet wird.

D.h.,-während gemäß der Lehre der DE-A 19 508 558 der Oxidationsstufe B Beschickungsgas zugeführt wird, das im wesentlichen nur aus Propylen, molekularem Sauerstoff und Propan besteht, wird der Oxidationsstufe B bei der erfindungsgemäßen Verfahrensweise ein Beschickungsgas zugeführt, das in notwendiger Weise Propylen, molekularen Sauerstoff, Propan und molekularen Stickstoff enthält. Die Auswahl der vorgenannten Bestandteile des Beschickungsgasgemisches der Oxidationsstufe B gewährleisten, daß im Rahmen der erfindungsgemäßen Verfahrensweise nicht nur ein Beschickungsgasgemisch der Oxidationsstufe B zur Anwendung kommt, dessen Sauerstoffgrenzkonzentration (bezüglich der Begriffsdefinition vgl.
DE-A 19 508 558) anwendungstechnisch in vollem Umfang zu befriedigen vermag, sondern das gleichzeitig eine Verringerung der Nebenproduktbildung an unerwünschtem Propionaldehyd und/oder unerwünschter Propionsäure erlaubt.

Selbstredend kann das Beschickungsgasgemisch der Oxidationsstufe B bei der erfindungsgemäßen Verfahrensweise neben den bereits genannten Bestandteilen zusätzlich noch andere Bestandteile wie z.B. CO, CO₂, H₂O, Edelgase wie He und/oder Ar, Wasserstoff, Methan, Ethylen, Ethan, Butane, Butene, Butine, Pentane, Propin, Allene, und/oder Acrolein enthalten. In der Regel sollte der Anteil des Beschickungsgasgemisches der Oxidationsstufe B an molekularem Stickstoff, bezogen auf die in diesem Beschickungsgasgemisch enthaltene Menge an Propylen, erfindungsgemäß nicht unter 5 mol-% betragen. D.h., beim erfindungsgemäßen Verfahren kann der Anteil des Beschickungsgasgemisches der Oxidationsstufe B an molekularem Stickstoff, bezogen auf die enthaltene Menge an Propylen wenigstens 10 mol-%, oder wenigstens 15 mol-% oder wenigstens 20 mol-%, oder wenigstens 25 mol-%, oder wenigstens 50 mol-%, aber auch wenigstens 100 mol-%, oder wenigstens 200 mol-%, oder wenigstens 500 mol-%, oder wenigstens 750 mol-%, oder wenigstens 1000 mol-% betragen. Im Normalfall wird das Verhältnis von im Beschickungsgasgemisch der Oxidationsstufe B enthaltener molarer Menge an molekularem Stickstoff zu im Beschickungsgasgemisch der Oxidationsstufe B enthaltener molarer Menge an Propylen erfindungsgemäß jedoch ≤ 40 : 1, häufig ≤ 30 : 1, vielfach ≤ 20 : 1 und oft ≤ 10 : 1 betragen. Günstig ist es, wenn beim erfindungsgemäßen Verfahren der Anteil des Beschickungsgasgemisches der Oxidationsstufe B an molekularem Stickstoff, bezogen auf die enthaltene Menge an Propylen, 600 mol-% bis 1600 mol-% beträgt.

Das molare Verhältnis von im Beschickungsgasgemisch der Oxidationsstufe B enthaltener Menge an molekularem Stickstoff zu im Beschickungsgasgemisch der Oxidationsstufe B enthaltener Menge an Propan wird beim erfindungsgemäßen Verfahren in der Regel nicht-kleiner als 0,05 betragen. Im Normalfall wird dieses Verhältnis aber auch nicht oberhalb von fünf liegen. D.h., erfindungsgemäß kann das molare Verhältnis von im Beschickungsgasgemisch der Oxidationsstufe B enthaltener Menge an molekularem Stickstoff zu im Beschickungsgasgemisch der Oxidationsstufe B enthaltener Menge an Propan 0,05 bis 5, oder 0,1 bis 4, oder 0,5 bis 3, oder 1 bis 2,5, oder ca. 2 betragen.

Häufig wird man beim erfindungsgemäßen Verfahren die Zusammensetzung des Beschickungsgasgemisches der Oxidationsstufe B so wählen, daß es die nachfolgenden molaren Verhältnisse erfüllt:
Propan : Propen : N₂ : O₂ : H₂O : sonstige
   = 0,5 bis 20 : 1 : 0,1 bis 40 : 0,1 bis 10 : 0 bis 20 : 0 bis 1.
Mit Vorteil betragen die vorgenannten molaren Verhältnisse erfindungsgemäß
   = 2 bis 10 : 1 : 0,5 bis 20 : 0,5 bis 5 : 0,01 bis 10 : 0 bis 1.
Günstig ist es auch, wenn die vorgenannten molaren Verhältnisse erfindungsgemäß
   = 3 bis 6 : 1 : 1 bis 10 : 1 bis 3 : 0,1 bis 2 : 0 bis 0,5 betragen.

Wesentliches Merkmal der erfindungsgemäßen Verfahrensweise ist, daß das Produktgasgemisch A der Stufe A, im Unterschied zum Fall einer homogenen und/oder heterogen katalysierten partiellen Oxidehydrierung von Propan, molekularen Wasserstoff enthält und daß vor der Verwendung des Produktgasgemisches A zur Beschickung des wenigstens einen Oxidationsreaktors der Stufe B aus dem Produktgasgemisch A im Unterschied zur Lehre der EP-A 117 146 wenigstens eine Teilmenge dieses molekularen Wasserstoff abgetrennt und, im Unterschied zur Lehre der DE-A 19 508 558, zur Minderung der Propionaldehyd- und/oder Propionsäurenebenproduktbildung, durch molekularen Stickstoff ersetzt wird.

In der Regel wird beim erfindungsgemäßen Verfahren das molare Verhältnis von im Produktgasgemisch A enthaltenem Propylen zu im Produktgasgemisch A enthaltenem molekularem Wasserstoff ≤ 100, üblicherweise ≤ 75, häufig ≤ 50, oft ≤ 40, vielfach ≤ 30, oder ≤ 25, oder ≤ 20 betragen.

D.h., erfindungsgemäße Verfahren sind insbesondere auch solche, bei denen das molare Verhältnis von im Produktgasgemisch A enthaltenem Propylen zu im Produktgasgemisch A enthaltenem molekularem Wasserstoff ≤ 15 oder ≤ 10 oder ≤ 5 oder ≤ 3 oder ≤ 2 oder ≤ 1 beträgt.

Im Normalfall wird der Reziprokwert des vorgenannten Verhältnisses 20 nicht überschreiten.

D.h., üblicherweise wird beim erfindungsgemäßen Verfahren das molare Verhältnis von im Produktgasgemisch A enthaltenem Propylen zu im Produktgasgemisch A enthaltenem molekularem Wasserstoff ≥ 0,05, meist ≥ 0,025, oft ≥ 0,1, häufig ≥ 0,25, vielfach ≥ 0,5 oder ≥ 0,75 oder auch ≥ 0,9 betragen.

Um in der Stufe A bei der erfindungsgemäß durchzuführenden partiellen heterogen katalysierten Dehydrierung, bezogen auf einmaligen Durchgang, interessante Umsätze zu erreichen, muß man in der Regel bei relativ hohen Reaktionstemperaturen arbeiten (in typischer Weise liegen diese Reaktionstemperaturen bei 300 bis 700°C) . Da die Dehydrierung (Spaltung von C-H) gegenüber der Crakkung (Spaltung von C-C) kinetisch benachteiligt ist, erfolgt sie an selektiv wirkenden Katalysatoren. Pro gebildetem Propylenmolekül wird dabei in der Regel ein Wasserstoffmolekül als Nebenprodukt erzeugt. Infolge der selektiv wirkenden Katalysatoren, die üblicherweise so beschaffen sind, daß sie unter Ausschluß von Sauerstoff bei den oben genannten Temperaturen (z.B. bei 600°C) eine signifikante Dehydrierung entfalten (bei Propan-Belastungen der Katalysatoren von z.B. 1000 h⁻¹ beträgt die Propylenausbeute im Regelfall wenigstens 30 mol-% im einmaligen Durchgang (bezogen auf eingesetztes Propan)), entstehen Nebenprodukte wie Methan, Ethylen und Ethan nur in untergeordneten Mengen.

Da die Dehydrierreaktion unter Volumenzunahme abläuft, kann der Umsatz durch Erniedrigung des Partialdrucks der Produkte gesteigert werden. Dies läßt sich in einfacher Weise z.B. durch Dehydrierung bei vermindertem Druck und/oder durch Zumischen von im wesentlichen inerten Verdünnungsgasen wie z.B. Wasserdampf erreichen, der für die Dehydrierreaktion im Normalfall ein Inertgas darstellt. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Außerdem kann Wasserdampf als Verdünnungsgas in der nachfolgenden Oxidationsstufe B mitverwendet werden. Wasserdampf läßt sich aber auch leicht teilweise oder vollständig aus dem Produktgasgemisch A des erfindungsgemäßen Verfahrens A abtrennen (z.B. durch Kondensieren), was die Möglichkeit eröffnet, bei der Weiterverwendung des dabei erhältlichen Produktgasgemisches A' in der Oxidationsstufe B den Anteil des erfindungswesentlich mitzuverwendenden Verdünnungsgases N₂ zu erhöhen. Dabei ist es erfindungsgemäß durchaus möglich, die Gesamtmenge oder auch nur eine Teilmenge des in der Oxidationsstufe B erfindungsgemäß mitzuverwendenden molekularen Stickstoff auch schon zur Verdünnung in der Stufe A mitzuverwenden. Weitere für die Stufe A geeignete Verdünnungsmittel sind z.B. CO, CO₂ und Edelgase wie He, Ne und Ar. Alle genannten Verdünnungsmittel können entweder für sich oder in Form unterschiedlichster Gemische in der Stufe A mitverwendet werden. Erfindungsgemäß von Vorteil ist, daß die für die Stufe A geeigneten Verdünnungsmittel in der Regel auch für die Oxidationsstufe B geeignete Verdünnungsmittel- sind. Generell sind sich in der jeweiligen Stufe inert verhaltende (d.h., zu weniger als 5 mol-%, bevorzugt zu weniger als 3 mol-% und noch besser zu weniger als 1 mol-% sich chemisch verändernde) Verdünnungsmittel bevorzugt. Prinzipiell kommen für die erfindungsgemäße Stufe A alle im Stand der Technik bekannten Dehydrierkatalysatoren in Betracht. Sie lassen sich grob in zwei Gruppen unterteilen. Nämlich in solche, die oxidischer Natur sind (z.B. Chromoxid und/oder Aluminiumoxid) und in solche, die aus wenigstens einem auf einem, in der Regel oxidischen, Träger abgeschiedenen, in der Regel vergleichsweise edlen, Metall (z.B. Platin) bestehen.

Unter anderem können damit für die erfindungsgemäße Stufe A alle Dehydrierkatalysatoren eingesetzt werden, die in der WO 99/46039, der US-A 4,788,371, der EP-A 705 136, der WO 99/29420, der US-A 5 220 091, der US-A 5 430 220, der US-A 5 877 369, der EP-A 117 146, der DE-A 19 937 106, der DE-A 19 937 105 sowie der DE-A 19 937 107 empfohlen werden. Im besonderen können für alle in dieser Schrift als für die erfindungsgemäße Stufe A geeignet angesprochenen Dehydrierverfahrensvarianten sowohl der Katalysator gemäß Beispiel 1, als auch gemäß Beispiel 2, als auch gemäß Beispiel 3, als auch gemäß Beispiel 4 der DE-A 19 937 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, daß die Summe der Gewichtsprozente 100 Gew.-% ergibt.

Zur Durchführung der Stufe A des erfindungsgemäßen Verfahrens kommen prinzipiell alle im Stand der Technik bekannten Reaktortypen und Verfahrensvarianten in Betracht. Beschreibungen solcher Verfaizrensvarianten enthalten z.B. alle bezüglich der Dehydrierkatalysatoren genannten Schriften des Standes der Technik.

Eine vergleichsweise ausführliche Beschreibung von erfindungsgemäß geeigneten Dehydrierverfahren enthält auch "Catalytica® Studies Division, Oxidative Dehydrogenation and Alternative Dehydrogenation Processes, Study Number 4192 OD, 1993, 430 Ferguson Drive, Mountain View, California, 94043-5272 U.S.A..

Charakteristisch für die partielle heterogen katalysierte Dehydrierung von Propan ist, daß sie endotherm verläuft. D.h., die für die Einstellung der erforderlichen Reaktionstemperatur benötigte Wärme (Energie) muß entweder dem Reaktionsgas vorab und/oder im Verlauf der katalytischen Dehydrierung zugeführt werden.

Ferner ist es für heterogen katalysierte Dehydrierungen von Propan aufgrund der hohen benötigten Reaktionstemperaturen typisch, daß in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann, wie bereits erwähnt, das zur katalytischen Dehydrierung bei erhöhter Temperatur über die Katalysatoroberfläche zu leitende Propan mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene-Kohlenstoff Verbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine Unterdrückung der Bildung von Kohlenstoffablagerungen ist aber auch dadurch möglich, daß man dem katalytisch zu dehydrierenden Propan, bevor es bei erhöhter Temperatur über den Dehydrierkatalysator geführt wird, molekularen Wasserstoff zusetzt.

Selbstverständlich besteht auch die Möglichkeit, Wasserdampf und molekularen Wasserstoff dem katalytisch zu dehydrierenden Propan im Gemisch zuzusetzen. Ein Zusatz von molekularem Wasserstoff zur katalytischen Dehydrierung von Propan mindert auch die unerwünschte Bildung von Allen und Acethylen als Nebenprodukten.

Eine geeignete Reaktorform für die erfindungsgemäße Stufe A ist der Festbettrohr- bzw. Rohrbündelreaktor. D.h., der Dehydrierkatalysator befindet sich in einem oder in einem Bündel von Reaktionsrohren als Festbett. Die Reaktionsrohre werden dadurch beheizt, daß im die Reaktionsrohre umgebenden Raum ein Gas, z.B. ein Kohlenwasserstoff wie Methan, verbrannt wird. Günstig ist es, diese direkte Form der Kontaktrohrerwärmung lediglich auf den ersten etwa 20 bis 30 % der Festbettschüttung anzuwenden und die verbleibende Schüttungslänge durch die im Rahmen der Verbrennung freigesetzte Strahlungswärme auf die erforderliche Reaktionstemperatur aufzuwärmen. Auf diesem Weg ist eine annähernd isotherme Reaktionsführung erreichbar. Geeignete Reaktionsrohrinnendurchmesser betragen etwa 10 bis 15 cm. Ein typischer Dehydrierrohrbündelreaktor umfaßt 300 bis 1000 Reaktionssrohre. Die Temperatur im Reaktionsrohrinneren bewegt sich im Bereich von 300 bis 700°C, vorzugsweise im Bereich von 400 bis 700°C. Mit Vorteil wird das Reaktionsgas dem Rohrreaktor auf die Reaktionstemperatur vorerwärmt zugeführt. Häufig verläßt das Produktgasgemisch das Reaktionsrohr mit einer 50 bis 100°C tiefergelegenen Temperatur. Im Rahmen der vorgenannten Verfahrensweise ist die Verwendung von oxidischen Dehydrierkatalysatoren auf der Grundlage von Chrom- und/oder Aluminiumoxid zweckmäßig. Häufig wird man kein Verdünnungsgas mitverwenden, sondern von im wesentlichen reinem Propan als Ausgangsreaktionsgas ausgehen. Auch der Dehydrierkatalysator wird meist unverdünnt angewandt.

Großtechnisch würde man etwa drei Rohrbündelreaktoren parallel betreiben, zwei dieser Reaktoren würden sich in der Regel im Dehydrierbetrieb befinden, während in einem der Reaktoren die Katalysatorbeschickung regeneriert wird.

Vorstehende Verfahrensweise wird beispielsweise beim in der Literatur bekannten BASF-Linde Propan-Dehydrierverfahren angewendet.

Desweiteren wird sie beim sogenannten "steam active reforming (STAR) process" angewendet, der von der Philips Petroleum Co. entwickelt wurde (vgl. z.B. US-A 4 902 849, US-A 4 996 387 und US-A 5 389 342). Als Dehydrierkatalysator wird im STAR-Prozeß mit Vorteil Promotoren enthaltendes Platin auf Zink (Magnesium) Spinel als Träger angewendet (vgl. z.B. US-A 5 073 662). Im Unterschied zum BASF-Linde Propan-Deyhdrierverfahren wird das zu dehydrierende Propan beim STAR-Prozeß mit Wasserdampf verdünnt. Typisch ist ein molares Verhältnis von Wasserdampf zu Propan im Bereich von 4 bis 6. Der Arbeitsdruck liegt häufig bei 3 bis 8 atm und die Reaktionstemperatur wird zweckmäßig zu 480 bis 620°C gewählt. Typische Katalysatorbelastungen mit dem totalen Reaktionsgasgemisch liegen bei 0,5 bis 10 h⁻¹.

-Selbstverständlich kann die erfindungsgemäße Stufe A auch im Wanderbett gestaltet werden. Beispielsweise kann das Katalysatorwanderbett in einem Radialstromreaktor untergebracht sein. In selbigem bewegt sich der Katalysator langsam von oben nach unten während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren hintereinander geschaltet zu betreiben (in typischer Weise bis zu vier). Dadurch lassen sich zu hohe Unterschiede der Temperaturen des Reaktionsgasgemisches am Reaktoreingang und am Reaktorausgang vermeiden (bei der adiabaten Betriebsweise fungiert das Reaktionsgasausgangsgemisch als Wärmeträger, von dessen Wärmeinhalt die Reaktionstemperatur abhängig ist) und trotzdem ansprechende Gesamtumsätze erzielen.

Wenn das Katalysatorbett den Wanderbettreaktor verlassen hat, wird es der Regenerierung zugeführt und anschließend wiederverwendet. Als Dehydrierkatalysator kann für dieses Verfahren z.B. ein kugelförmiger Dehydrierkatalysator eingesetzt werden, der im wesentlichen aus Platin auf kugelförmigem Aluminiumoxidträger besteht. Bei der UOP-Variante wird dem zu dehydrierenden Propan Wasserstoff zugefügt, um eine vorzeitige Katalysatoralterung zu vermeiden. Der Arbeitsdruck liegt typisch bei 2 bis 5 atm. Das Wasserstoff zu Propan Verhältnis (das molare) beträgt zweckmäßig 0,1 bis 1. Die Reaktionstemperaturen liegen bevorzugt bei 550 bis 650°C und die Katalysatorbelastung mit Reaktionsgasgemisch wird zu etwa 2 bis 6 h⁻¹ gewählt.

Bei den beschriebenen Festbettverfahren kann die Katalysatorgeometrie ebenfalls kugelförmig, aber auch zylindrisch (hohl oder voll) sein.

Als weitere Verfahrensvariante für die erfindungsgemäße Stufe A beschreibt Proceedings De Witt, Petrochem. Review, Houston Texas, 1992 a, N1 die Möglichkeit einer heterogen katalysierten Propandehydrierung im Wirbelbett, bei der das Propan nicht verdünnt wird.

Zweckmäßigerweise werden dabei zwei Wirbelbetten nebeneinander betrieben, von denen sich eines in der Regel im Zustand der Regenerierung befindet. Als Aktivmasse kommt dabei Chromoxid auf Aluminiumoxid zum Einsatz. Der Arbeitsdruck beträgt typisch 1 bis 1,5 atm und die Dehydriertemperatur liegt in der Regel bei 550 bis 600°C. Die für die Dehydrierung erforderliche Wärme wird dadurch in das Reaktionssystem eingebracht, daß der Dehydrierkatalysator auf die Reaktionstemperatur vorerhitzt wird. Der -Arbeitsdruck liegt regelmäßig bei 1 bis 2 atm und die Reaktionstemperatur beträgt typisch 550 bis 600°C. Die vorstehende Dehydrierweise ist in der Literatur auch als Snamprogetti-Yarsintez Verfahren bekannt.

Alternativ zu den vorstehend beschriebenen Verfahrensweisen kann die erfindungsgemäße Stufe A auch nach einem von ABB Lummus Crest entwickelten Verfahren realisiert werden (vgl. Proceedings De Witt, Petrochem. Review, Houston Texas, 1992, P1).

Den bisher beschriebenen heterogen katalysierten Dehydrierverfahren des Propans ist gemein, daß sie bei Propanumsätzen von > 30 mol-% (in der Regel ≤ 60 mol-%) betrieben werden (bezogen auf einmaligen Reaktordurchgang).

Erfindungsgemäß von Vorteil ist es, daß es für das erfindungsgemäße Verfahren ausreichend ist, in der Stufe A einen Propanumsatz von ≥ 5 mol-% bis ≤ 30 mol-% oder ≤ 25 mol-% zu erzielen. D.h., erfindungsgemäß kann die Stufe A auch bei Propanumsätzen von 10 bis 20 mol-% betrieben werden (die Umsätze beziehen sich auf einmaligen Reaktordurchgang). Dies rührt unter anderem daher, daß die verbliebene Menge an nicht umgesetztem Propan in der nachfolgenden Oxidationsstufe B mit molekularem Stickstoff verdünnt wird, was die Propionaldehyd- und/oder Propionsäurenebenproduktbildung mindert.

Für die Realisierung der vorgenannten Propanumsätze ist es günstig, die erfindungsgemäße Propandehydrierung in der Stufe A bei einem Arbeitsdruck von 0,3 bis 2 atm durchzuführen. Ferner ist es günstig, das zu dehydrierende Propan mit Wasserdampf zu verdünnen. So ermöglicht die Wärmekapazität des Wassers einerseits einen Teil der Auswirkung der Endothermie der Dehydrierung auszugleichen und andererseits reduziert die Verdünnung mit Wasserdampf den Edukt- und Produktpartialdruck, was sich günstig auf die Gleichsgewichtslage der Dehydrierung auswirkt. Ferner wirkt sich die Wasserdampfmitverwendung, wie bereits erwähnt, vorteilhaft auf die Standzeit des Dehydrierkatalysators aus. Bei Bedarf kann als weiterer Bestandteil auch molekularer Wasserstoff zugegeben werden. Das molare Verhältnis von molekularem Wasserstoff zu Propan ist dabei in der Regel ≤ 5. Das molare Verhältnis von Wasserdampf zu Propan kann demnach bei der Stufe-A-Variante mit vergleichsweise geringem Propanumsatz ≥ 0 bis 30, zweckmäßig 0,1 bis 2 und günstig 0,5 bis 1 betragen. Als günstig für eine Verfahrensweise mit niederem Propanumsatz erweist es sich auch, daß bei einmaligem Reaktordurchgang des Reaktionsgases lediglich eine vergleichsweise niedrige Wärmemenge verbraucht wird und zur Umsatzerzielung bei einmaligem Reaktordurchgang vergleichsweise niedrige Reaktionstemperaturen ausreichend sind.

Es ist daher erfindungsgemäß zweckmäßig bei der Stufe-A-Variante mit vergleichsweise geringem Propanumsatz die Propandehydrierung (quasi) adiabat durchzuführen. D.h., man wird das Reaktionsgasausgangsgemisch in der Regel auf eine Temperatur von 500 bis 700°C erhitzen (z.B. durch Direktbefeuerung der es umgebenden Wandung), bzw. auf 550 bis 650°C. Im Normalfall wird dann ein einziger adibater Durchgang durch ein Katalysatorbett ausreichend sein, um den gewünschten Umsatz zu erzielen, wobei sich das Reaktionsgasgemisch um etwa 30°C bis 200°C (je nach Umsatz) abkühlen wird. Ein Beisein von Wasserdampf als Wärmeträger macht sich auch unter dem Gesichtspunkt einer adiabaten Fahrweise vorteilhaft bemerkbar. Die niedrigere Reaktionstemperatur ermöglicht längere Standzeiten des verwendeten Katalysatorbetts.

Prinzipiell ist auch die erfindungsgemäße Stufe-A-Variante mit vergleichsweise geringem Propanumsatz, ob adiabatisch oder isotherm gefahren, sowohl in einem Festbettreaktor als auch in einem Wanderbett- oder Wirbelbettreaktor durchführbar.

Bemerkenswerterweise ist zu ihrer Realisierung, insbesondere im adiabatischen Betrieb, ein einzelner Schachtofenreaktor als Festbettreaktor ausreichend, der von Reaktionsgasgemisch axial und/oder radial durchströmt wird.

Im einfachsten Fall handelt es sich dabei um ein einziges Reaktionsrohr dessen Innendurchmesser 0,1 bis 10 m, eventuell auch 0,5 bis 5 m beträgt und in welchem das Katalysatorfestbett auf einer Trägervorrichtung (z.B. ein Gitterrost) aufgebracht ist. Das mit Katalysator beschickte Reaktionsrohr, das im adiabaten Betrieb wärmeisoliert ist, wird dabei mit dem heißen, Propan enthaltenden, Reaktionsgas axial durchströmt. Die Katalysatorgeometrie kann dabei sowohl kugelförmig oder ringförmig sein. In vorteilhafter Weise ist der Katalysator im vorgenannten Fall aber auch splittförmig anwendbar. Zur Verwirklichung einer radialen Strömung des Propan enthaltenden Reaktionsgases kann der Reaktor z.B. aus zwei in einer Mantelhülle befindlichen, zentrisch ineinander gestellten, zylindrischen Gitterrosten bestehen und die Katalysatorschüttung in deren Ringspalt angeordnet sein. Im adiabaten Fall wäre die Mantelhülle wiederum thermisch isoliert.

Als Katalysatorbeschickung für die erfindungsgemäße Stufe-A-Variante mit Vergleichsweise geringem Propanumsatz bei einmaligem Durchgang eignen sich insbesondere die in der DE-A 19 937 107 of- fenbarten, vor allem alle beispielhaft offenbarten, Katalysatoren.

Nach längerer Betriebsdauer sind vorgenannte Katalysatoren z.B. in einfacher Weise dadurch regenerierbar, daß man bei einer Temperatur von 300 bis 600°C, häufig bei 400 bis 500°C, zunächst in ersten Regenerierungsstufen mit Stickstoff verdünnte Luft über das Katalysatorbett leitet. Die Katalysatorbelastung mit Regeneriergas kann dabei z.B. 50 bis 10000 h⁻¹ und der Sauerstoffgehalt des Regeneriergases 0,5 bis 20 Vol.-% betragen.

In nachfolgenden weiteren Regenerierungstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (z.B. N₂) zu spülen.

Anschließend ist es in der Regel empfehlenswert, noch mit reinem molekularem Wasserstoff oder mit durch Inertgas verdünntem molekularem Wasserstoff (der Wasserstoffgehalt sollte ≥ 1 Vol.-% betragen) im ansonsten gleichen Bedingungsraster zu regenerieren.

Die erfindungsgemäße Stufe A-Variante mit vergleichsweise geringem Propanumsatz (≤ 30 mol-%) kann in allen Fällen bei den gleichen Katalysatorbelastungen (sowohl das Reaktionsgas insgesamt, als auch das in selbigem enthaltene Propan betreffend) betrieben werden wie die Varianten mit hohem Propanumsatz (> 30 mol-%) . Diese Belastung mit Reaktionsgas kann z.B. 100 bis 10000 h⁻¹, häufig 100 bis 3000 h⁻¹, d.h., vielfach ca. 100 bis 2000 h⁻¹ betragen.

In besonders eleganter Weise läßt sich die erfindungsgemäße Stufe A-Variante mit vergleichsweise geringem Propanumsatz in einem Hordenreaktor verwirklichen.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8 aber auch 4 bis 6 betragen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinander gestellten zylindrischen Gitterrosten angeordnet.

In zweckmäßiger weise wird das Reaktionsgasgemisch auf seinem Weg von einem Katalysatorbett zum nächsten Katalysatorbett, z.B. durch Überleiten über mit heißen Gasen erhitzte Wärmetauscherrippen oder durch Leiten durch mit heißen Brenngasen erhitzte Rohre, im Hordenreaktor einer Zwischenerhitzung unterworfen.

Wird der Hordenreaktor im übrigen adiabat betrieben, ist es für die gewünschten Propanumsätze (≤ 30 mol-%) insbesondere bei Verwendung der in der DE-A 19 937 107 beschriebenen Katalysatoren, insbesondere der beispielhaften Ausführungsformen, ausreichend, das Reaktionsgasgemisch auf eine Temperatur von 450 bis.550⁰C vorerhitzt in den Dehydrierreaktor zu führen und innerhalb des Hordenreaktors in diesem Temperaturbereich zu halten. D.h., die gesamte Propandehydrierung ist so bei äußerst niederen Temperaturen zu verwirklichen, was sich für die Standzeit der Katalysatorfestbetten als besonders günstig erweist.

Noch geschickter ist es, die vorstehend geschilderte Zwischenerhitzung auf direktem Weg durchzuführen. Dazu wird dem Reaktionsgasgemisch entweder bereits vor Durchströmung des ersten Katalysatorbettes und/oder zwischen den nachfolgenden Katalysatorbetten in begrenztem Umfang molekularer Sauerstoff zugesetzt. Je nach verwendetem Dehydrierkatalysator wird so eine begrenzte Verbrennung der im Reaktionsgasgemisch enthaltenen Kohlenwasserstoffe, gegebenenfalls bereits auf der Katalysatoroberfläche abgeschiedener Kohle bzw. kohleähnlicher Verbindungen und/oder von im Verlauf der Propandehydrierung gebildetem und/oder dem Reaktionsgasgemisch zugesetztem Wasserstoff bewirkt (es kann auch anwendungstechnisch zweckmäßig sein, im Hordenreaktor Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen z.B. jene der Schriften US-A 4 788 371, US-A 4886928, US-A 5430209, US-A 55 530 171, US-A 5 527 979 und US-A 5 563 314 in Betracht); beispielsweise könnten solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender und im wesentlichen konstanter Temperatur möglich, was besonders lange Standzeiten ermöglicht.

In der Regel sollte eine wie vorstehend beschriebene Sauerstoffeinspeisung so vorgenommen werden, daß der Sauerstoffgehalt des Reaktionsgasgemisches, bezogen auf die darin enthaltene Menge an Propan und Propylen, 0,5 bis 10 Vol.-% beträgt. Als Sauerstoffquelle kommen dabei sowohl reiner molekularer Sauerstoff oder mit Inertgas, z.B. CO, CO₂, N₂, Edelgase, verdünnter Sauerstoff, insbesondere aber auch Luft in Betracht. Die resultierenden Verbrennungsgase wirken in der Regel zusätzlich verdünnend und fördern dadurch die heterogen katalysierte Propandehydrierung.

Die Isothermie der heterogen katalysierten Propandehydrierung läßt sich dadurch weiter verbessern, daß man im Hordenreaktor in den.Räumen zwischen den Katalysatorbetten geschlossene, vor ihrer Füllung evakuierte, Einbauten (z.B. rohrförmige), anbringt. Selbstredend können derartige Einbauten auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit das Reaktionsgasgemisch in der Stufe A des erfindungsgemäßen Verfahren auf die benötigte Reaktionstemperatur zu erwärmen besteht auch darin, einen Teil des darin enthaltenen Propan und/oder H₂ mittels molekularem Sauerstoff zu verbrennen (z.B. an geeigneten spezifisch wirkenden Verbrennungskatalysatoren, z.B. durch einfaches Überleiten und/oder Durchleiten) und mittels der so freigesetzten Verbrennungswärme die Erwärmung auf die gewünschte Reaktionstemperatur zu bewirken. Die resultierenden Verbrennungsprodukte wie CO₂, H₂O sowie das den für die Verbrennung benötigten molekularen Sauerstoff gegebenenfalls begleitende N₂ bilden vorteilhaft inerte Verdünnungsgase.

Erfindungsgemäß wesentlich ist, daß das in der Stufe A verwendete Propan kein reines Propan sein muß. Vielmehr kann das verwendete Propan bis zu 50 Vol.-% anderer Gase wie z.B. Ethan, Methan, Ethylen, Butane, Butene, Propin, Acethylen, H₂S, SO₂, Pentane etc. enthalten. Zweckmäßig enthält das einzusetzende Rohpropan wenigstens 60 Vol.-%, vorteilhaft wenigstens 70 Vol.-%, bevorzugt wenigstens 80 Vol.-%, besonders bevorzugt wenigstens 90 Vol.-% und ganz besonders bevorzugt wenigstens 95 Vol.-% an Propan. Insbesondere kann für die erfindungsgemäße Stufe A auch ein Gemisch aus Propan, Propylen und aus der Oxidationsstufe herrührendem Kreisgas verwendet werden.

Das im Rahmen des erfindungsgemäßen Verfahrens die Stufe A verlassende Produktgasgemisch A enthält wenigstens die Bestandteile "Propan, Propen und molekularen Wasserstoff. Darüber hinaus wird es in der Regel aber auch noch Gase aus der Gruppe umfassend N₂, H₂O, Methan, Ethan, Ethylen, CO und CO₂ enthalten.

Es wird sich in der Regel bei einem Druck von 0,3 bis 10 atm befinden und häufig eine Temperatur von 400 bis 550°C, in günstigen Fällen von 450 bis 500°C aufweisen.

Erfindungsgemäß wesentlich ist, wenigstens einen Teil des im Produktgasgemisch A enthaltenen Wasserstoff abzutrennen, bevor das dabei erhältliche Produktgasgemisch A' in der Stufe B zur Beschickung des wenigstens einen Oxidationsreaktors verwendet wird. Dies kann z.B. dadurch erfolgen, daß man das Produktgasgemisch A, gegebenenfalls nachdem man es zuvor in einem indirekten Wärmetauscher abgekühlt hat (zweckmäßiger Weise wird die dabei entnommene Wärme zum Erhitzen eines für das erfindungsgemäße Verfahren benötigten feed-Gases verwendet) über eine, in der Regel zu einem Rohr gestaltete, Membran leitet, die lediglich für den molekularen Wasserstoff durchlässig ist. Der so abgetrennte molekulare Wasserstoff kann bei Bedarf teilweise in die Stufe A rückgeführt oder einer sonstigen Verwertung zugeführt werden. Im einfachsten Fall kann er in Brennstoffzellen verbrannt werden.

Alternativ dazu kann die wenigstens teilweise erforderliche Wasserstoffabtrennung auch durch partielle Kondensation, Adsorption und/oder Rektifikation (vorzugsweise unter Druck) vorgenommen werden.

In der Regel wird man erfindungsgemäß wenigstens 10 mol-% oder wenigstens 25 mol-%, häufig wenigstens 35 mol-% oder wenigstens 50 mol-%, vielfach wenigstens 75 mol-% und oft die Gesamtmenge des im Produktgasgemisch A enthaltenen molekularen Wasserstoff abtrennen, bevor es als Produktgasgemisch A' in der Stufe B erfindungsgemäß zur Beschickung des wenigstens einen Oxidationsreaktor eingesetzt wird.

Selbstredend kann bei Bedarf im Rahmen der Abtrennung von molekularem Wasserstoff auch eine Abtrennung anderer, von Propan und Propylen verschiedener, Bestandteile des Produktgasgemisches A vorgenommen werden.

Eine einfache Möglichkeit, im wesentlichen alle von Propan und Propylen verschiedenen Bestandteile des Produktgasgemisches A abzutrennen, besteht darin, das, vorzugsweise abgekühlte (vorzugsweise-auf Temperaturen von 10 bis 70°C), Produktgasgemisch A, z.B. bei einem Druck von 0,1 bis 50 atm und einer Temperatur von 0 bis 100°C, mit einem (vorzugsweise hochsiedenden) organischen Lösungsmittel (vorzugsweise ein hydrophobes), in welchem Propan und Propen bevorzugt absorbiert werden, in Kontakt zu bringen (z.B. durch einfaches Durchleiten). Durch nachfolgende Desorption, Rektifikation und/oder Strippung mit einem bezüglich der erfindungsgemäßen Oxidationsstufe B sich inert verhaltenden Gas und/oder molekularem Sauerstoff (z.B. Luft) werden das Propan und Propen im Gemisch rückgewonnen und zur Beschickung des wenigstens einen Oxidationsreaktors der Stufe B verwendet. Das den molekularen Wasserstoff enthaltende Abgas der Absorption kann man z.B. wieder einer Membrantrennung unterwerfen und dann, bei Bedarf, den abgetrennten Wasserstoff in der Stufe A mitverwenden. Vorzugsweise sollte der Siedepunkt des organischen Absorptionsmittels ≥ 100°C, besonders bevorzugt ≥ 180°C betragen. Die Absorption kann sowohl in Kolonnen als auch in Rotationsabsorbern vorgenommen werden. Dabei kann im Gleichstrom oder im Gegenstrom gearbeitet werden. Geeignete Absorptionskolonnen sind z.B. Bodenkolonnen (mit Glocken-, Zentrifugal- und/oder Siebböden), Kolonnen mit strukturierten Packungen (z.B. Blechpackungen mit einer spezifischen Oberfläche von 100 bis 500 m²/m³, z.B. Mellapak® 250 Y) und Füllkörperkolonnen (z.B. mit Raschig-Füllkörpern gefüllte). Selbstverständlich kommen aber auch Riesel- und Sprühtürme, Graphitblockabsorber, Oberflächenabsorber wie Dickschichtund Dünnschichtabsorber sowie Rotationskolonnen, Tellerwäscher, Kreuzschleierwäscher und Rotationswäscher in Betracht.

Erfindungsgemäß günstig ist es, wenn das zu verwendende organische Absorptionsmittel einerseits die bereits gegebene Empfehlung für den Siedepunkt erfüllt, andererseits gleichzeitig aber ein nicht zu hohes Molekulargewicht aufweist. Mit Vorteil beträgt das Molekulargewicht des Absorptionsmittels ≤ 300 g/mol.

Erfindungsgemäß geeignete Absorptionsmittel sind z.B. relativ unpolare organische Lösungsmittel, die vorzugsweise keine nach außen wirkende polare Gruppe enthalten. Beispielhaft genannt seien aliphatische (z.B. C₈- bis C₁₈-Alkane) oder aromatische Kohlenwasserstoffe, z.B. Mittelölfraktionen aus der Paraffindestillation, oder Ether mit sperrigen Gruppen am O-Atom, oder Gemische davon, wobei diesen ein polares Lösungsmittel wie z.B. das in der DE-A 4 308 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, z.B. 4-Methyl-4'-benzyl-diphenylmethan und dessen Isomere 2-Methyl-2'-benzyl-diphenyl-methyl, 2-Methyl-4'-benzyl-diphenylethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew. -% Diphenyl (Biphenyl) und etwa 75 Gew.-% Diphenylether, beispielsweise das im Handel erhältliche Diphyl®. Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Erwähnt werden sollen als mögliche Absorptionsmittel auch Octane, Nonane, Decane, Undecane, Dodecane, Tridecane, Tetradecane, Pentadecane, Hexadecane, Heptadecane und Octadecane.

Abschließend sei darauf hingewiesen, daß die Teil- oder Vollabtrennung des molekularen Wasserstoff aus dem Produktgasgemisch A auch durch selektive heterogen katalysierte Verbrennung desselben mit molekularem Sauerstoff vorgenommen werden kann. Diesbezüglich geeignete Katalysatoren offenbaren beispielsweise die US-A 4 788 371, US-A 4 886 928, US-A 5 430 209, US-A 55 530 171, US-A 5 527 979 und US-A 5 563 314.

Nach der erfindungsgemäß erfolgten wenigstens Teilabtrennung des im Produktgasgemisch A enthaltenen molekularen Wasserstoff kann das dabei erhaltene Produktgasgemisch A' in der erfindungsgemäß erforderlichen zweiten Stufe B zur Beschickung wenigstens eines Oxidationsreaktors verwendet und in dem wenigstens einen Oxidationsreaktor das Propylen einer selektiven heterogen katalysierten Gasphasen-Partialoxidation mit molekularem Sauerstoff zu einem Acrolein und/oder Acrylsäure enthaltenden Produktgasgemisch B verwendet werden. Bei Bedarf wird dabei das Produktgasgemisch A' vorab durch indirekten Wärmetausch auf die in dem wenigstens einen Oxidationsreaktor erforderliche Reaktionstemperatur gebracht.

Prinzipiell läuft die heterogen katalysierte Gasphasen-Partialoxidation von Propylen zu Acrylsäure mit molekularem Sauerstoff in zwei längs der Reaktionskoordinate aufeinanderfolgenden Schritten ab, von denen der erste zum Acrolein und der zweite vom Acrolein zur Acrylsäure führt.

Dieser Reaktionsablauf in zwei zeitlich aufeinanderfolgenden Schritten eröffnet in an sich bekannter Weise die Möglichkeit, die Stufe B des erfindungsgemäßen Verfahrens in zwei hintereinander angeordneten Oxidationszonen auszuführen, wobei in jeder der beiden Oxidationszonen der zu verwendende oxidische Katalysator in optimierender Weise angepaßt werden kann. So wird für die erste Oxidationszone (Propylen → Acrolein) in der Regel ein Katalysator auf der Basis von die Elementkombination Mo-Bi-Fe enthaltenden Multimetalloxiden bevorzugt, während für die zweite Oxidationszone (Acrolein- Acrylsäure) normalerweise Katalysatoren auf der Basis von die Elementkombination Mo-V enthaltenden Multimetalloxiden bevorzugt werden.

Entsprechende Multimetalloxidkatalysatoren für die beiden Oxidationszonen sind vielfach vorbeschrieben und dem Fachmann wohlbekannt. Beispielsweise verweist die EP-A 253 409 auf Seite 5 auf entsprechende US-Patente.

Günstige Katalysatoren für die beiden Oxidationszonen offenbaren auch die DE-A 4 431 957 und die DE-A 4431949. Dieses gilt insbesondere für jene der allgemeinen Formel I in den beiden vorgenannten Schriften. In der Regel wird das Produktgemisch aus der ersten Oxidationszone ohne Zwischenbehandlung in die zweite Oxidationszone überführt.

Die einfachste Realisierungsform der beiden Oxidationszonen bildet daher ein Rohrbündelreaktor innerhalb dessen sich die Katalysatorbeschickung längs der einzelnen Kontaktrohre mit Beendigung des ersten Reaktionsschrittes entsprechend ändert (derartige als Stufen B erfindungsgemäß geeignete Propylenpartialoxidationen lehren z.B. die EP-A 911313, die EP-A 979813, die EP-A 990636 und die DE-A 2830765). Gegebenenfalls wird dabei die Beschickung der Kontaktrohre mit Katalysator durch eine Inertschüttung unterbrochen.

Vorzugsweise werden die beiden Oxidationszonen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet werden. Während die Kontaktrohre in der Regel von einem Wärmeträger umspült werden, erreicht dieser eine wie vorstehend beschrieben angebrachte Inertschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationsstufe verläßt, zu mindern. Anstelle von Rohrbündelreaktoren können auch Plattenwärmetauscherreaktoren mit Salz- und/oder Siede-Kühlung, wie sie z.B. die DE-A 19 929 487 und die DE-A 19 952 964 beschreiben, eingesetzt werden.

Die Reaktionsstemperatur in der ersten Oxidationszone liegt in der Regel bei 300 bis 450°C, bevorzugt bei 320 bis 390°C. Die Reaktionstemperatur in der zweiten Oxidationszone liegt in der Regel bei 200 bis 300°C, häufig bei 220 bis 290°C. Der Reaktionsdruck beträgt in beiden Oxidationszonen zweckmäßig 0,5 bis 5, vorteilhaft 1 bis 3 atm. Die Belastung (N1/1-h) der Oxidationskatalysatoren mit Reaktionsgas beträgt in beiden Oxidationszonen häufig 1500 bis 2500 h⁻¹ bzw. bis 4000 h⁻¹.

Prinzipiell können die beiden Oxidationszonen beim erfindungsgemäßen Verfahren so gestaltet werden, wie es z.B. in der DE-A 19 837 517, der DE-A 19 910 506, der DE-A 19 910 508 sowie der DE-A 19 837 519 beschrieben ist. Üblicherweise wird die externe Temperierung in den beiden Oxidationszonen, gegebenenfalls in Mehrzonenreaktorsystemen, in an sich bekannter Weise an die spezielle Reaktionsgasgemischzusammensetzung sowie Katalysatorbeschickung angepaßt.

Der für die erfindungsgemäß erforderliche Stufe B als Oxidationsmittel insgesamt benötigte molekulare Sauerstoff kann dem Beschickungsgasgemisch der Stufe B in seiner Gesamtmenge vorab zugegeben werden. Selbstverständlich kann aber auch nach der ersten Oxidationszone mit Sauerstoff ergänzt werden.

Vorzugsweise wird in der ersten Oxidationszone ein molares Verhältnis Propylen : molekularer Sauerstoff von 1 : 1 bis 3, häufig 1 : 1,5 bis 2 eingestellt. Die gleichen numerischen Werte eignen sich für das molare Verhältnis Acrolein: molekularer Sauerstoff in der zweiten Oxidationszone.

In beiden Oxidationszonen wirkt sich ein Überschuß an molekularem Sauerstoff in der Regel vorteilhaft auf die Kinetik der Gasphasenoxidation aus. Im Unterschied zu den Verhältnissen in der erfindungsgemäß anzuwendenden Stufe A werden die thermodynamischen Verhältnisse durch das molare Reaktandenverhältnis im wesentlichen nicht beeinflußt, da die heterogen katalysierte Gasphasen-Partialoxidation des Propylens zu Acrylsäure kinetischer Kontrolle unterliegt. Prinzipiell kann daher z.B. in der ersten Oxidationszone auch das Propylen gegenüber dem molekularen Sauerstoff im molaren Überschuß vorgelegt werden. In diesem Fall kommt dem überschüssigen Propylen faktisch die Rolle eines Verdünnungsgases zu.

Prinzipiell ist die heterogen katalysierte Gasphasen-Partialoxidation von Propylen zu Acrylsäure aber auch in einer einzigen Oxidationszone realisierbar. In diesem Fall erfolgen beide Reaktionsschritte in einem Oxidationsreaktor der mit einem Katalysator beschickt ist, der die Umsetzung beider Reaktionsschritte zu katalysieren vermag. Selbstredend kann sich auch die Katalysatorbeschickung innerhalb der Oxidationszone längs der Reaktionskoordinate kontinuierlich oder abrupt ändern. Natürlich kann bei einer Ausführungsform der erfindungsgemäß mitzuverwendenden Stufe B in Gestalt zweier hintereinandergeschalteter Oxidationszonen aus dem das die erste Oxidationszone verlassenden Produktgasgemisch in selbigem enthaltenes, in der ersten Oxidationszone als Nebenprodukt entstandenes, Kohlenoxid und Wasserdampf bei Bedarf vor der Weiterleitung in die zweite Oxidationszone teilweise oder vollständig abgetrennt werden. Vorzugsweise wird man erfindungsgemäß eine Verfahrensweise wählen, die solch einer Abtrennung nicht bedarf.

Als Quelle für den in der Oxidationsstufe B benötigten molekularen Sauerstoff, der dem Produktgasgemisch A' vor dessen Verwendung zur Beschickung der Oxidationsstufe B zugemischt wird, kommen sowohl reiner molekularer Sauerstoff als auch mit Inertgas wie CO₂, CO, Edelgasen, N₂ und/oder gesättigten Kohlenwasserstoffen verdünnter molekularer Sauerstoff in Betracht.

In zweckmäßiger Weise wird man wenigstens zur Deckung eines Teilbedarfs an molekularem Sauerstoff Luft als Sauerstoffquelle verwenden, da auf diese Weise der in der Stufe B erfindungsgemäß mitzuverwendende molekulare Stickstoff ins Reaktionssystem eingebracht werden kann.

Mit Vorteil wird beim erfindungsgemäßen Verfahren das Produktgasgemisch A' im wesentlichen nur aus Propan und Propylen bestehen (der Anteil an davon verschiedenen Bestandteilen beträgt zweckmäßig ≤ 5 Vol.-% bzw. ≤ 2 Vol.-%) und als Quelle für molekularen Sauerstoff für die nachfolgende Stufe B wird ausschließlich Luft verwendet.

Durch Zudosieren von kalter Luft zu heißem Produktgasgemisch A' kann im Rahmen des erfindungsgemäßen Verfahrens auch auf direktem Weg eine Abkühlung des Produktgasgemisches A' bewirkt werden.

Ist Acrolein das Zielprodukt, wird man in der Stufe B die zweite Oxidationszone in zweckmäßiger Weise nicht mehr anwenden.

Das die erfindungsgemäß anzuwendende Stufe B verlassende Produktgasgemisch B ist in der Regel im wesentlichen zusammengesetzt aus dem Zielprodukt Acrolein oder Acrylsäure oder dessen Gemisch mit - Acrolein, nicht umgesetztem molekularem Sauerstoff, Propan, molekularem Stickstoff, als Nebenprodukt entstandenem und/oder als Verdünnungsgas mitverwendetem Wasserdampf, als Nebenprodukt und/oder als Verdünnungsgas mitverwendeten Kohlenoxiden, sowie geringen Mengen sonstiger niederer Aldehyde, Kohlenwasserstoffe und anderer inerter Verdünnungsgase.

Das Zielprodukt kann aus dem Produktgasgemisch B in an sich bekannter Weise abgetrennt werden (z.B. durch partielle Kondensation der Acrylsäure oder durch Absorption von Acrylsäure in Wasser oder in einem hochsiedenden hydrophoben organischen Lösungsmittel oder durch Absorption von Acrolein in Wasser oder in wäßrigen Lösungen niederer Carbonsäuren sowie anschließende Aufarbeitung der Absorbate; alternativ kann das Produktgasgemisch auch fraktioniert kondensiert werden; vgl. z.B. EP-A 117146, DE-A 4308087, DE-A 4335172, DE-A 4436243, DE-A 19 924 532 sowie DE-A 19 924 533).

Nicht umgesetztes Propylen und/oder Acrolein werden gegebenenfalls gleichfalls abgetrennt und in die Stufe B rückgeführt.

Ansonsten können die von Acrylsäure und Acrolein verschiedenen wesentlichen Bestandteile des nach der Zielproduktabtrennung verbleibenden Restgases je nach Bedarf und verwendetem Dehydrierkatalysator jeweils für sich abgetrennt und/oder mit dem Propan als Kreisgas in die Dehydierstufe A rückgeführt werden, um dort, wie beschrieben, den Dehydrierumsatz zu beeinflussen. Selbstverständlich kann aber auch das nicht umgesetzte Propan im Gemisch mit dem nicht umgesetzten Propylen für sich in die Stufe A rückgeführt werden. Bei kontinuierlicher Ausführung des erfindungsgemäßen Verfahrens erfolgt so eine kontinuierliche Umsetzung von Propan zu Acrylsäure und/oder Acrolein.

Die Abtrennung von Propan und Propen aus dem nach der Zielproduktabtrennung verbleibende Restgas (es enthält in der Regel O₂, CO, CO₂, H₂O, N₂, Edelgase sowie sonstiger niederer Aldehyde und Kohlenwasserstoffe) kann, wie bereits beschrieben, durch Absorption mit nachfolgender Desorption und/oder Strippung (sowie Absorptionsmittelwiederverwendung) in einem hochsiedenden hydrophoben organischen Lösungsmittel erfolgen. Weitere Trennmöglichkeiten sind Adsorption, Rektifikation und partielle Kondensation.

Bei Verwendung von Dehydrierkatalysatoren, die gegenüber Sauerstoff oder Sauerstoff enthaltenden Verbindungen empfindlich sind, wird man diese Oxygenate vor einer Rückführung von Kreisgas in die Stufe A aus dem Kreisgas abtrennen. Eine solche Sauerstoffabtrennung kann auch sinnvoll sein um eine Oxidation des Propans in -der Dehydrierstufe A zu vermeiden. Die Dehydrierkatalysatoren der DE-A 19 937 107 sind nicht empfindlich gegen Oxygenate (insbesondere jene gemäß Beispiel 1 bis 4 der DE-A).

Eine andere Abtrennmöglichkeit bietet, wie gleichfalls bereits erwähnt, die fraktionierte Destillation. Vorzugsweise wird eine fraktionierte Druckdestillation bei tiefen Temperaturen durchgeführt. Der anzuwendende Druck kann z.B. 10 bis 100 bar betragen. Als Rektifikationskolonnen können Füllkörperkolonnen, Bodenkolonnen oder Packungskolonnen eingesetzt werden. Als Bodenkolonnen eignen sich solche mit Dual-Flow-Böden, Glockenböden oder Ventilböden. Das Rücklaufverhältnis kann z.B. 1 bis 10 betragen. Andere Trennmöglichkeiten bilden z.B. Druckextraktion, Druckwechseladsorption, Druckwäsche, partielle Kondensation und Druckextraktion.

Selbstverständlich kann erfindungsgemäß auch die Gesamtmenge an Restgas in die Stufe A rückgeführt werden. In diesem Fall kann sich der Auslaß für von Propan, Propen und molekularem Sauerstoff verschiedenen Gasbestandteilen ausschließlich zwischen dem Produktgemisch A und dem Produktgemisch A' befinden.

Selbstredend kann ein weiterer Auslaß nach der Zielproduktabtrennung eingerichtet sein. Falls das in die Propandehydrierung rückgeführte Kreisgas Kohlenmonoxid enthält, kann dieses, bevor mit frischem Propan ergänzt wird, katalytisch zu CO₂ verbrannt werden. Die dabei freigesetzte Reaktionswärme kann zum Aufheizen auf die Dehydriertemperatur Anwendung finden.

Eine katalytische Nachverbrennung von im Restgas enthaltenem CO zu CO₂ kann auch dann empfehlenswert sein, wenn eine Abtrennung der Kohlenoxide aus dem Restgas vor dessen Rückführung als Kreisgas in die Propandehydrierung angestrebt wird, läßt sich doch CO₂ vergleichsweise einfach abtrennen (z.B. durch Wäsche mit einer basischen Flüssigkeit).

Natürlich kann auch so verfahren werden, daß man einen Teil des Restgases unverändert in die Propandehydrierung rückführt und nur aus dem verbliebenen Teil Propan und Propen im Gemisch abtrennt und ebenfalls in die Propandehydrierung und/oder in die Stufe B rückführt. Im letzteren Fall vereint man den verbliebenen Teil des Restgases zweckmäßigerweise mit dem Produktgasgemisch A.

Im Rahmen einer fraktionierten Destillation des Restgases kann die Trennlinie z.B. so gelegt werden, daß am Kopf der Rektifikationskolonne im wesentlichen alle diejenigen Bestandteile abgetrennt werden, deren Siedepunkt tiefer als der Siedepunkt von Propen liegt. Diese Bestandteile werden in erster Linie die Kohlenoxide CO und CO₂ sowie nicht umgesetzter Sauerstoff und Ethylen sowie Methan und N₂ sein.

Häufig wird das erfindungsgemäße Verfahren so durchgeführt, daß im Produktgasgemisch B wenigstens 70 mol-%, vorzugsweise wenigstens 80 mol-% des in den verschiedenen Reaktionsstufen insgesamt zugeführten molekularen Sauerstoff umgesetzt worden sind.

Vorzugsweise wird beim erfindungsgemäßen Verfahren in der zweiten Oxidationszone der Stufe B bei einem molaren Acrolein : molekularem Sauerstoff : Wasserdampf : Propan : molekularem Stickstoff : sonstige Verdünnungsgase Verhältnis von 1 : 0,5 bis 1 : 0,1 bis 1 : 0,5 bis 6 : 1 bis 10 : 0 bis 5 gearbeitet.

Der erfindungsgemäße Vorteil einer verminderten Propionaldehydund/oder Propionsäurenebenproduktbildung tritt im wesentlichen unabhängig davon ein, welche Multimetalloxidkatalysatoren in der erfindungsgemäß mitzuverwendenden Stufe B eingesetzt werden. Er ist im wesentlichen auch unabhängig davon vorhanden, ob die volumenspezifische Katalysatoraktivität in der Stufe B konstant gehalten oder längs der Reaktionskoordinate zunehmend gewählt wird.

Insbesondere tritt der erfindungsgemäße Vorteil dann auf, wenn in der ersten Oxidationszone der Stufe B Multimetalloxidkatalysatoren verwendet werden, die jenen der allgemeinen Formel I oder II oder III aus der DE-A 19 910 506 entsprechen und wenn in der zweiten Oxidationszone der Stufe B Multimetalloxidkatalysatoren verwendet werden, die jenen der allgemeinen Formel I oder I' oder II aus der DE-A 19 910 508 entsprechen.

Als erfindungsgemäß geeignete Katalysatorgeometrien kommen erfindungsgemäß für die erste bzw. zweite Oxidationszone der erfindungsgemäß anzuwendenden Stufe B diejenigen in Betracht, die die DE-A 19 910 506 bzw. die DE-A 19 910 508 empfehlen.

Ferner können die für die erfindungsgemäße Stufe B empfohlenen Rohrbündelreaktoren, was die Stromführung von Reaktionsgas und Temperiermedium (z.B. Salzbad) anbetrifft, sowohl im Gleichstrom als auch im Gegenstrom betrieben werden. Selbstredend können auch Querstromführungen überlagert werden. Besonders günstig ist eine mäanderförmige Führung des Temperiermediums um die Kontaktrohre herum. die über den Reaktor betrachtet wiederum im Gleichstrom oder im Gegenstrom zum Reaktionsgasgemisch erfolgen kann.

In der Regel werden für die erfindungsgemäße Stufe A Reaktoren mit passivierten Innenwandungen eingesetzt. Die Passivierung kann z.B. dadurch erfolgen, daß auf die Innenwandung vorab der Dehydrierung gesintertes Aluminiumoxid aufgebracht wird.
Sie kann aber auch in situ durch Zusatz geringer Mengen passivierender Hilfsstoffe (z.B. Sulfide) zum Reaktionsgasgemisch bewirkt werden.

Erfindungsgemäß kann auch so verfahren werden, daß in Zeiten, in denen die Katalysatorbeschickung der Propandehydrierung regeneriert wird, die Acrylsäureanlage mit Propen aus einer anderen Quelle (z.B. Cracker, Pipeline, Tank) versorgt wird. In diesem Fall ist kein zusätzlicher Dehydrierreaktor für Regenerierphasen erforderlich.

Abschließend sei festgehalten, daß das erfindungsgemäße Verfahren auch solche Verfahren umfassen soll, bei denen wenigstens ein Teil des in der Stufe A gebildeten Wasserstoff in die Stufe A integriert quasi in situ kontinuierlich entfernt (z.B. verbrannt) wird, z.B. durch Oxidation zu Wasserdampf mittels eines zusätzlich zum Dehydrierkatalysator zugesetzten reduzierbaren Metalloxids, wie es z.B. die EP-A 832 056 beschreibt.

### Beispiele

### A) Herstellung eines Multimetalloxidkatalysators für die erste Oxidationszone der Stufe B

### 1. Herstellung einer Ausgangsmasse 1

In 775 kg einer wäßrigen salpetersauren Wismutnitratlösung (11,2 Gew.-% Bi, freie Salpetersäure 3 bis 5 Gew.-%; Massendichte: 1,22 bis 1,27 g/ml) wurden bei 25°C portionsweise 209,3 kg Wolframsäure (72,94 Gew.-% W) eingerührt. Das resultierende wäßrige Gemisch wurde anschließend noch 2 h bei 25°C gerührt und anschließend sprühgetrocknet.

Die Sprühtrocknung erfolgte in einem Drehscheibensprühturm im Gegenstrom bei einer Gaseintrittstemperatur von 300 ± 10°C und einer Gasaustrittstemperatur von 100 ± 10°C. Das erhaltene Sprühpulver wurde anschließend bei einer Temperatur im Bereich von 780 bis 810°C calciniert (im luftdurchströmten Drehrohrofen (1,54 m³ Innenvolumen, 200 Nm³ Luft/h)). Wesentlich bei der genauen Einstellung der Calcinationstemperatur ist, daß sie an der angestrebten Phasenzusamensetzung des Calcinationsprodukts orientiert ⁻zu erfolgen hat. Gewünscht sind die Phasen WO₃ (monoklin) und Bi₂W₂O₉, unerwünscht ist das Vorhandensein von γ-Bi₂WO₆ (Russellit). Sollte daher nach der Calcination die Verbindung γ-Bi₂WO₆ anhand eines Reflexes im Pulverröntgendiffraktogramm bei einem Reflexwinkel von 2⊖ = 28,4° (CuKα-Strahlung) noch nachweisbar sein, so ist die Präparation zu wiederholen und die Calcinationstemperatur innerhalb des angegebenen Temperaturbereichs zu erhöhen, bis das Verschwinden des Reflexes erreicht wird. Das so erhaltene vorgebildete calcinierte Mischoxid wurde gemahlen, so daß der X₅₀-Wert (vgl.. Ullmann's Encyclopedia of Industrial Chemistry, 6^{th} Edition (1998) Electronic Release, Kapitel 3.1.4 oder DIN 66141) der resultierenden Körnung 5 µm betrug. Das Mahlgut wurde dann mit 1 Gew.-% (bezogen auf das Mahlgut) feinteiligem SiO₂ (Rüttelgewicht 150 g/l; X₅₀-Wert der SiO₂-Partikel betrüg 10 µm, die BET-Oberfläche betrug 100 m²/g) vermischt.

### 2. Herstellung einer Ausgangsmasse 2

Eine Lösung A wurde hergestellt, indem man bei 60°C unter Rühren in 600 1 Wasser 213 kg Ammoniumheptamolybdat löste und die resultierende Lösung unter Aufrechterhaltung der 60°C und Rühren mit 0,97 kg einer 20°C aufweisenden wäßrigen Kaliumhydroxidlösung (46,8 Gew.-% KOH) versetzte.

Eine Lösung B wurde hergestellt, indem man bei 60°C in 262,9 kg einer wäßrigen Cobaltnitratlösung (12,4 Gew.-% Co) 116,25 kg einer wäßrigen Eisennitratlösung (14,2 Gew.-% Fe) eintrug. Anschließend wurde unter Aufrechterhaltung der 60°C die Lösung B über einen Zeitraum von 30 Minuten kontinuierlich in die vorgelegte Lösung A gepumpt. Anschließend wurde 15 Minuten bei 60°C gerührt. Dann wurden dem resultierenden wäßrigen Gemisch 19,16 kg eines Kieselgels (46,80 Gew.-% SiO₂, Dichte: 1,36 bis 1,42 g/ml, pH 8,5 bis 9,5, Alkaligehalt max. 0,5 Gew.-%) zugegeben und danach noch weitere 15 Minuten bei 60°C gerührt.

Anschließend wurde in einem Drehscheibensprühturm im Gegenstrom sprühgetrocknet (Gaseintrittstemperatur: 400 ± 10°C, Gasaustrittstemperätur: 140± 5°C). Das resultierende Sprühpulver wies einen Glühverlust von ca. 30 Gew.-% auf (3 h bei 600°C glühen).

### 3. Herstellung der Multimetalloxidaktivmasse und des Katalysators

Die Ausgangsmasse 1 wurde mit der Ausgangsmasse 2 in der für eine Multimetalloxidaktivmasse der Stöchiometrie

[Bi₂W₂O₉ · 2WO₃]_{0,5}[MC)₁₂CO_{5,5}Fe_{2,94}Si_{1,59}K_{0,08}Oₓ]₁

erforderlichen Menge homogen vermischt. Bezogen auf die vorgenannte Gesamtmasse wurden zusätzlich 1,5 Gew.-% feinteiliges Graphit (Siebanalyse: min. 50 Gew.-% < 24 µm, max. 10 Gew.-% > 24 µm und < 48 µm, max. 5 Gew.-% > 48 µm, BET-Oberfläche: 6 bis 13 m²/g) homogen eingemischt. Das resultierende Trockengemisch wurde zu Hohlzylindern mit 3 mm Länge, 5 mm Außendurchmesser und 1,5 mm Wandstärke verpreßt und anschließend wie folgt thermisch behandelt.

Im Luft durchströmten Muffelofen (60 1 Innenvolumen, 1 N1/h Luft 5 pro Gramm Aktivmassevorläufermasse) wurde mit einer Aufheizrate von 180°C/h zunächst von Raumtemperatur (25°C) auf 190°C aufgeheizt. Diese Temperatur wurde für 1 h aufrechterhalten und dann mit einer Aufheizrate von 60°C/h auf 210°C erhöht. Die 210°C wurden wiederum während 1 h aufrechterhalten, bevor sie mit einer Aufheizrate von 60°C/h auf 230°C erhöht wurde. Diese Temperatur wurde ebenfalls 1 h aufrechterhalten bevor sie, wiederum mit einer Aufheizrate von 60°C/h, auf 265°C erhöht wurde. Die 265°C wurden anschließend ebenfalls während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten. Dabei resultierten für die erste Oxidationszone der Stufe B geeignete Vollkatalysatorringe V.

### B) Herstellung eines Multimetalloxidkatalysators für die zweite Oxidationszone der Stufe B

### 1. Herstellung der katalytisch aktiven Oxidmasse Mo₁₂V₃W_{1,2}Cu_{2,4}Oₙ

190 g Kupfer(II)acetatmonohydrat wurden in 2700 g Wasser zu einer Lösung I gelöst. In 5500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und anschließend soviel einer 25gew.-%igen wäßrigen NH₃-Lösung zugesetzt, bis wieder eine Lösung entstand. Diese wurde bei einer Austrittstemperatur von 110°C sprühgetrocknet. Das resultierende Sprühpulver wurde je kg Pulver mit 0,25 kg einer 30gew.-%igen wäßrigen Essigsäurelösung mit einem Kneter der Fa. Werner & Pfleiderer vom Typ ZS1-80 verknetet und anschließend bei einer Temperatur von 110⁰C während 10 h im Trockenschrank getrocknet.

700 g des so erhaltenen Katalysatorvorläufers wurden in einem - Luft/Stickstoffgemisch [(200 N1 N₂/15 N1 Luft)/h] in einem Drehrohrofen (50 cm lang, 12 cm Innendurchmesser) calciniert. Im Rahmen der Calcinierung wurde die Knetmasse zunächst innerhalb von einer Stunde von Raumtemperatur (ca. 25°C) kontinuierlich auf 325⁰C erhitzt. Anschließend wurde während 4 h auf dieser Temperatur gehalten. Dann wurde innerhalb von 15 min auf 400°C erwärmt, bei dieser Temperatur während 1 h gehalten und dann auf Raumtemperatur abgekühlt.

Das calcinierte katalytisch aktive Material wurde zu einem feinteiligen Pulver gemahlen, von dem 50 % der Pulverpartikel ein Sieb der Maschenweite 1 bis 10 µm passierten und dessen Anteil an Partikel mit einer Längstausdehnung oberhalb von 50 µm weniger als 1 % betrug.

### 2. Schalenkatalysatorherstellung

28 kg ringförmiger Trägerkörper (7 mm Außendurchmesser, 3 mm Länge, 4 mm Innendurchmesser, Steatit, mit einer Oberflächenrauhigkeit Rz gemäß EP-B 714700 von 45 µm und mit einem auf das Volumen der Trägerkörper bezogenen Porengesamtvolumen ≤ 1 Vol.-%, Hersteller: Ceramtec DE) wurden in einem Dragierkessel (Neigungswinkel 90°; Hicoater der Fa. Lödige, DE) von 200 1 Innenvolumen gefüllt. Anschließend wurde der Dragierkessel mit 16 U/min in Rotation versetzt. Über eine Düse wurden innerhalb von 25 min 2000 g einer aus 75 Gew.-% H₂O und 25 Gew.-% Glycerin bestehenden wäßrigen Lösung auf die Trägerkörper aufgesprüht. Gleichzeitig wurden im selben Zeitraum 7,35 kg des katalytisch aktiven Oxidpulvers aus a) über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nicht beobachtet. Nach beendeter Zugabe von Pulver und wäßriger Lösung wurde bei einer Drehgeschwindigkeit von 2 Umdrehungen/min 20 min. 110°C heiße Luft in den Dragierkessel geblasen. Anschließend wurde noch 2 h bei 250°C in ruhender Schüttung (Hordenofen) unter Luft getrocknet. Es wurden ringförmige, für die zweite Oxidationszone der Stufe B geeignete, Schalenkatalysatoren S erhalten, deren Anteil an oxidischer Aktivmasse, bezogen auf die Gesamtmasse, 21 Gew.-% betrug. Die Schalendicke lag, sowohl über die Oberfläche eines Trägerkörpers als auch über die Oberfläche verschiedener Trägerkörper betrachtet, bei 240 ± 25 µm.

### C) Beschickung eines für die Stufe B geeigneten Reaktionsrohrsystems R und dessen Temperierung

### 1. Beschickung eines ersten Reaktionsrohres

Ein Reaktionsrohr (V2A Stahl; 30 mm Außendurchmesser; 2 mm Wandstärke; 26 mm Innendurchmesser, Länge: 439 cm, sowie ein in der Reaktionsrohrmitte zentriertes Thermorohr (6 mm Außendurchmesser) zur Aufnahme eines Thermoelements mit dem die Temperatur im Reaktionsrohr ermittelt werden kann) wird von unten nach oben auf einem Kontaktstuhl (32 cm Länge) zunächst auf einer Länge von 30 cm mit eine rauhe Oberfläche aufweisenden Steatitkugeln (4 bis 5 mm Durchmesser; Inertmaterial zum Erwärmen des Reaktionsgasausgangsgemisches) beschickt. Daran schließt sich eine 100 cm lange Beschickung an, die aus einem homogenen Gemisch aus 373 g Vollkatalysatorringen V aus A) sowie 160 g an Steatitringen der Geometrie 5 mm x 3 mm x 2 mm (Außendurchmesser x Länge x Wandstärke) besteht. Abgeschlossen wird die Reaktionsrohrbeschickung von zunächst einer 170 cm langen Schüttung aus Vollkatalysatorringen V aus A) und daran anschließend einer 107 cm langen Schüttung aus den bereits genannten, eine rauhe Oberfläche aufweisenden Steatitkugeln (4 bis 5 mm Durchmesser).

### 2. Beschickung eines zweiten Reaktionsrohres

Ein Reaktionsrohr (V2A Stahl; 30 mm Außendurchmesser; 2 mm Wandstärke; 26 mm Innendurchmesser, Länge: 441 cm, sowie ein in der Reaktionsrohrmitte zentriertes Thermo-Rohr (6 mm Außendurchmesser) zur Aufnahme eines Thermoelements mit dem die Temperatur im Reaktionsrohr ermittelt werden kann) wird von unten nach oben auf einem Kontaktstuhl (34 cm Länge) zunächst auf einer Länge von 200 cm mit dem Schalenkatalysator S aus B) und daran anschließend auf einer Länge von 100 cm mit einem Schalenkatalysator S'(hergestellt wie der Schalenkatalysator S, der Anteil der oxidischen Aktivmasse, bezogen auf die Gesamtmasse, wird jedoch zu nur 17 Gew.-% gewählt) beschickt. Eine 56 cm lange Beschickung mit eine rauhe Oberfläche aufweisenden Steatitkugeln (4 bis 5 mm Durchmssser) schließt die Reaktionsrohrbeschickung ab.

### 3. Verbindungsrohr

Das erste und das zweite Reaktionsrohr werden an ihrem, dem jeweiligen Kontaktstuhl gegenüberliegenden, Ende durch ein Verbindungsrohr (V2A Stahl; 1 m Länge; Innendurchmesser 9 mm) miteinander verbunden. Das Verbindungsrohr weist in der Mitte die Möglichkeit auf, ein molekularen Sauerstoff enthaltenes Gas zu ergänzen.

### 4. Temperierung des Reaktionsrohrsystems

Zur Temperierung der Reaktionsrohre werden gepumpte Salzschmelzen verwendet. Die gepumpte Salzschmelze für das erste Reaktionsrohr wird auf einer Temperatur von X⁰C gehalten. Die gepumpte Salzschmelze für das zweite Reaktionsrohr wird auf einer Temperatur von Y⁰C gehalten. Das Verbindungsrohr wird auf einer Temperatur von 200°C gehalten (mittels elektrischer Heizmatten).

### D) Durchführung von Gasphasenoxidationen

1. Zusammensetzung eines Reaktionsgasgemisches A:

| | |
|---|---|
| 5,5 Vol-% | Propen, |
| 9, 7 Vol-% | O₂, |
| 10 Vol.-% | H₂O und |
| 74,8 Vol. -% | N₂. |

2. Zusammensetzung eines Reaktionsgasgemisches B:

| | |
|---|---|
| 5,5 Vol-% | Propen, |
| 9, 7 Vol-% | O₂, |
| 10 Vol.-% | H₂O und |
| 74,8 Vol.-% | Propan. |

3. Zusammensetzung eines Reaktionsgasgemisches C:

| | |
|---|---|
| 5, 5 Vol-% | Propen, |
| 9,7 Vol-% | O₂, |
| 10 Vol.-% | H₂O und |
| 37,8 Vol.-% | Propan und |
| 37, 0 Vol.-% | N₂. |

Das Reaktionsrohrsystem aus C) wird jeweils mit dem Reaktionsgeasgemisch A bzw. mit dem Reaktionsgasgemisch B bzw. mit dem Reaktionsgasgemisch C beschickt. Die Zufuhr des Reaktionsgemisches erfolgt in das erste Reaktionsrohr und zwar in das den Kontaktstuhl aufweisende Rohrende.

Die Belastung der Katalysatorbeschickung des ersten Reaktionsrohres wird jeweils zu 100 N1 Propen/1 Kat·h (h⁻¹) gewählt. In das Verbindungsrohr werden in allen drei Fällen 75 N1/h an Raumtemperatur aufweisendem molekularen Sauerstoff ergänzt.

Die Thermostatisierung des Reaktionsrohrsystems wird so gewählt, daß in allen drei Fällen, bezogen auf einmaligen Durchgang, im - ersten Reaktionsrohr ein Umsatz des Propens von 94 mol-% und im zweiten Reaktionsrohr ein Umsatz des im ersten Reaktionsrohr gebildeten Acroleins von 97,5 mol-% resultiert.

Nach einer Betriebsdauer von 10 h wird das das zweite Reaktionsrohr verlassende Produktgasgemisch jeweils auf seinen Gehalt an Propionsäure (bezogen auf den Gesamtgehalt an Acrylsäure) analysiert.

Die nachfolgende Tabelle zeigt die Ergebnisse.
1. Reaktionsgasgemisch A
   X = 328°C
   Y = 270°C
   Gehalt an Propionsäure: 0,02 mol-%.
2. Reaktionsgasgemisch B
   X = 358°C
   Y = 295°C
   Gehalt an Propionsäure: 0,14 mol-%.
3. Reaktionsgasgemisch C
   X = 343°C
   Y = 281°C
   Gehalt an Propionsäure: 0,08 mol-%.

D.h.,. die Propionsäurenebenproduktbildung ist im Fall B mehr als sechs mal so groß als im Fall A, was ausweist, daß eine Verwendung von molekularem Stickstoff als Verdünnungsgas die Propionsäurenebenproduktbildung zu mindern vermag.

### E) Katalytische Nachverbrennung von CO zu CO₂ in einem Produktgasgemisch B), aus welchem die Zielprodukte bereits abgetrennt sind

Ein Gasgemisch der Zusammensetzung 2,5 Vol.-% CO, 1 Vol.-% Propen, 82 Vol.-% Propan und 14,5 Vol.-% eines Gemisches aus H₂O, N₂ und CO₂ wurde bei einer Temperatur von 212°C (die Temperierung erfolgte mittels auf dem Reaktionsrohr aufliegendem Aluminiumblock der mittels elektrischer Heizmatten temperiert wurde) nach Zusatz einer, auf das CO bezogenen, 2-fach stöchiometrischen Menge an molekularem O₂ durch eine Festbettschüttung (Rohrinnendurchmesser: 20,5 mm, zentriert im Rohr eine 4 mm Außendurchmesser aufweisenden Thermohülse für ein Innenthermoelement, Schüttlänge: 19 cm) des kommerziell erhältlichen BASF-Edelmetall-Katalysators RO 20 (Kugeln mit 3 mm Durchmesser, Al₂O₃ als Träger, Pd als Edelmetallaktivkomponente) geleitet (bei einer Katalysatorbelastung mit Gasgemisch von 1680 h⁻¹). Von dem im Gasgemisch enthaltenen CO wurden 94 mol-% zu CO₂ verbrannt. Die übrigen Gasbestandteile blieben im wesentlichen unverändert. Eine nachfolgende Abtrennung von Propan und Propen vom Gasgemisch durch fraktionierte Druckdestillation ist nun einfacher durchführbar.

## Patentansprüche

1. Verfahren zur Herstellung von Acrolein oder Acrylsäure oder deren Gemisch aus Propan, bei dem man
A) in einer ersten Stufe A das Propan einer partiellen heterogen katalysierten Dehydrierung in der Gasphase unter Bildung eines Produktgasgemisches A, das molekularen Wasserstoff, Propylen und nicht umgesetztes Propan enthält, unterwirft,
B) aus dem molekularen Wasserstoff, Propylen und nicht umgesetztes Propan enthaltenden Produktgasgemisch A der Stufe A von den darin enthaltenen, von Propan und Propylen verschiedenen, Bestandteilen wenigstens eine Teilmenge des molekularen Wasserstoff abtrennt und es dann als Produktgasgemisch A' in einer zweiten Stufe B zur Beschickung wenigstens eines Oxidationsreaktors verwendet und in dem wenigstens einen Oxidationsreaktor das Propylen einer selektiven heterogen katalysierten Gasphasen-Partialoxidation mit molekularem Sauerstoff zu einem Produktgasgemisch B, das Acrolein oder Acrylsäure oder deren Gemisch als Zielprodukt enthält, unterwirft, und
C) aus dem im Rahmen der partiellen Oxidation des Propylens in der Stufe B anfallenden Produktgasgemisch B in einer dritten Stufe C Zielprodukt abtrennt und wenigstens im Produktgasgemisch der Stufe B enthaltenes nicht umgesetztes Propan in die Dehydrierungsstufe A zurückführt,
**dadurch gekennzeichnet, daß** im Rahmen der partiellen Oxidation des Propylens in der Stufe B molekularer Stickstoff als Verdünnungsgas mitverwendet wird.

2. Verfahren nach Anspruch 1, **dadurch gekennzeichnet, daß** das Beschickungsgasgemisch des wenigstens einen Oxidationsreaktors in der zweiten Stufe B, bezogen auf darin enthaltendes Propylen, wenigstens 5 mol-% an molekularem Stickstoff enthält.

3. Verfahren nach Anspruch 1, **dadurch gekennzeichnet, daß** das Beschickungsgasgemisch des wenigstens einen Oxidationsreaktors in der zweiten Stufe B, bezogen auf darin enthaltendes Propylen, wenigstens 50 mol-% an molekularem Stickstoff enthält.

4. Verfahren nach Anspruch 1, **dadurch gekennzeichnet, daß** das Beschickungsgasgemisch des wenigstens einen Oxidationsreaktors in der zweiten Stufe B, bezogen auf darin enthaltendes Propylen, wenigstens 100 mol-% an molekularem Stickstoff enthält.

5. Verfahren nach einem der Ansprüche 1 bis 4, **dadurch gekennzeichnet, daß** das molare Verhältnis von im Beschickungsgasgemisch des wenigstens einen Oxidationsreaktors in der zweiten Stufe B enthaltener Menge an molekularem Stickstoff zu im selben Beschickungsgasgemisch enthaltener Menge an Propan wenigstens 0,05 beträgt.

6. Verfahren nach einem der Ansprüche 1 bis 4, **dadurch gekennzeichnet, daß** das molare Verhältnis von im Beschickungsgasgemisch des wenigstens einen Oxidationsreaktors in der zweiten Stufe B enthaltener Menge an molekularem Stickstoff zu im selben Beschickungsgasgemisch enthaltener Menge an Propan 0,05 bis 5 beträgt.

7. Verfahren nach einem der Ansprüche 1 bis 4, **dadurch gekennzeichnet, daß** das molare Verhältnis von im Beschickungsgasgemisch des wenigstens einen Oxidationsreaktors in der zweiten Stufe B enthaltener Menge an molekularem Stickstoff zu im selben Beschickungsgasgemisch enthaltener Menge an Propan 0,5 bis 3 beträgt.

8. Verfahren nach Anspruch 1, **dadurch gekennzeichnet, daß** die Zusammensetzung des Beschickungsgasgemisches des wenigstens einen Oxidationsreaktors in der zweiten Stufe B die nachfolgenden molaren Verhältnisse erfüllt:
Propan : Propen : N₂ : O₂ : H₂O : sonstige
= 0,5 bis 20 : 1 : 0,1 bis 40 : 0,1 bis 10 : 0 bis 20 : 0 bis 1.

9. Verfahren nach Anspruch 1, **dadurch gekennzeichnet, daß** die Zusammensetzung des Beschickungsgasgemisches des wenigstens einen Oxidationsreaktors in der zweiten Stufe B die nachfolgenden molaren Verhältnisse erfüllt:
Propan. : Propen : N₂ : O₂ : H₂O : sonstige
= 2 bis 10 : 1 : 0,5 bis 20 : 0,5 bis 5 : 0,01 bis 10 : 0 bis 1.

10. Verfahren nach Anspruch 1, **dadurch gekennzeichnet, daß** die Zusammensetzung des Beschickungsgasgemisches des wenigstens einen Oxidationsreaktors in der zweiten Stufe B die nachfolgenden molaren Verhältnisse erfüllt:
Propan : Propen : N₂ : O₂ : H₂O : sonstige
= 3 bis 6 : 1 : 1 bis 10 : 1 bis 3 : 0,1 bis 2 : 0 bis 0,5.

11. Verfahren nach einem der Ansprüche 1 bis 10, **dadurch gekennzeichnet, daß** das molare Verhältnis von im Produktgasgemisch A enthaltenem Propylen zu im Produktgasgemisch A enthaltenem molekularem Wasserstoff ≤ 100 beträgt.

12. Verfahren nach einem der Ansprüche 1 bis 10, **dadurch gekennzeichnet, daß** das molare Verhältnis von im Produktgasgemisch A enthaltenem Propylen zu im Produktgasgemisch A enthaltenem molekularem Wasserstoff ≤ 50 beträgt.

13. Verfahren nach einem der Ansprüche 1 bis 10, **dadurch gekennzeichnet, daß** das molare Verhältnis von im Produktgasgemisch A enthaltenem Propylen zu im Produktgasgemisch A enthaltenem molekularem Wasserstoff ≤ 10 beträgt.

14. Verfahren nach einem der Ansprüche 1 bis 13, **dadurch gekennzeichnet, daß** das molare Verhältnis von im Produktgasgemisch A enthaltenem Propylen zu im Produktgasgemisch A enthaltenem molekularem Wasserstoff ≥ 0,05 beträgt.

15. Verfahren nach einem der Ansprüche 1 bis 14, **dadurch gekennzeichnet, daß** der in der Stufe A erzielte Propanumsatz, bezogen auf einfachen Durchgang, 5 bis 25 mol-% beträgt.

16. Verfahren nach einem der Ansprüche 1 bis 14, **dadurch gekennzeichnet, daß** der in der Stufe A erzielte Propanumsatz, bezogen auf einfachen Durchgang, 10 bis 20 mol-% beträgt.

17. Verfahren nach Anspruch 15 oder 16, **dadurch gekennzeichnet, daß** das zu dehydrierende Propan in der Stufe A mit Wasserdampf verdünnt wird.

18. Verfahren nach Anspruch 17, **dadurch gekennzeichnet, daß** das molare Verhältnis von Wasserdampf zu zu dehydrierendem Propan 0,1 bis 2 beträgt.

19. Verfahren nach Anspruch 15 oder 16, **dadurch gekennzeichnet, daß** dem zu dehydrierenden Propan molekularer Wasserstoff zugesetzt wird.

20. Verfahren nach Anspruch 19, **dadurch gekennzeichnet, daß** das molare Verhältnis von molekularem Wasserstoff zu zu dehydrierendem Propan > 0 und ≤ 5 beträgt.

21. Verfahren nach einem der Ansprüche 15 bis 20, **dadurch gekennzeichnet, daß** die partielle heterogen katalysierte Dehydrierung des Propans in der Stufe A adiabat durchgeführt wird.

22. Verfahren nach einem der Ansprüche 15 bis 21, **dadurch gekennzeichnet, daß** die partielle heterogen katalysierte Dehydrierung des Propans in der Stufe A in einem axial oder radial durchströmten Festbettreaktor durchgeführt wird.

23. Verfahren nach einem der Ansprüche 15 bis 21, **dadurch gekennzeichnet, daß** die partielle heterogen katalysierte Dehydrierung des Propans in der Stufe A in einem Hordenreaktor durchgeführt wird.

24. Verfahren nach Anspruch 23, **dadurch gekennzeichnet, daß** die Temperatur des Reaktionsgasgemisches im gesamten Hordenreaktor 450 bis 550°C beträgt.

25. Verfahren nach Anspruch 23 oder 24, **dadurch gekennzeichnet, daß** der Hordenreaktor räumlich aufeinanderfolgend 2 bis 8 Katalysatorbetten enthält.

26. Verfahren nach einem der Ansprüche 23 bis 25, **dadurch gekennzeichnet, daß** dem Reaktionsgasgemisch während der partiellen heterogen katalysierten Dehydrierung des Propans in der Stufe . A molekularer Sauerstoff zugesetzt wird.

27. verfahren nach einem der Ansprüche 1 bis 26, **dadurch gekennzeichnet, daß** man aus dem Produktgasgemisch A vor seiner Weiterverwendung als Produktgasgemisch A' die enthaltene Menge an molekularem Wasserstoff abtrennt.

28. Verfahren nach einem der Ansprüche 1 bis 26, **dadurch gekennzeichnet, daß** man aus dem Produktgasgemisch A vor seiner Weiterverwendung als Produktgasgemisch A' die von Propan und Propylen verschiedenen Bestandteile abtrennt.

29. Verfahren nach einem der Ansprüche 1 bis 26, **dadurch gekennzeichnet, daß** man aus dem Produktgasgemisch A die von Propan und Propylen verschiedenen Bestandteile dadurch abtrennt, daß man das Produktgasgemisch A mit einem organischen Lösungsmittel in Kontakt bringt, in selbigem das Propan und Propylen selektiv absorbiert, durch nachfolgende Desorption wieder freisetzt und als Produktgasgemisch A' zur Beschickung des wenigstens einen Oxidationsreaktors in der Stufe B verwendet.

30. Verfahren nach einem der Ansprüche 1 bis 29, **dadurch gekennzeichnet, daß** man zur Beschickung des wenigstens einen Oxidationsreaktors der Stufe B Luft mitverwendet.

31. Verfahren nach einem der Ansprüche 1 bis 30, **dadurch gekennzeichnet, daß** man aus dem Produktgasgemisch B darin enthaltenes Acrolein und/oder Acrylsäure abtrennt, und von dem dabei verbleibenden Restgas wenigstens eine Teilmenge in die Dehydrierungsstufe A rückführt.

32. Verfahren nach Anspruch 28, **dadurch gekennzeichnet, daß** man aus dem Produktgasgemisch B darin enthaltenes Acrolein und/oder Acrylsäure abtrennt und die Gesamtmenge des dabei verbleibenden Restgases in die Dehydrierungsstufe A rückführt.

33. Verfahren nach einem der Ansprüche 1 bis 30, **dadurch gekennzeichnet, daß** man aus dem Produktgasgemisch B zunächst darin enthaltenes Acrolein und/oder Acrylsäure abtrennt, aus dem dabei verbleibenden Restgas die von Propan und Propylen verschiedenen Bestandteile abtrennt und das verbleibende Propan und Propen in die Dehydrierungsstufe A rückführt.

34. Verfahren nach einem der Ansprüche 1 bis 33, **dadurch gekennzeichnet, daß** die Stufe B aus zwei hintereinandergeschalteten Rohrbündelreaktoren besteht.

35. Verfahren nach Anspruch 23, **dadurch gekennzeichnet, daß** der Hordenreaktor wenigstens ein Katalysatorbett umfaßt, das die Verbrennung von Wasserstoff katalysiert.

36. Verfahren nach Anspruch 1, dadurch gekenzeichnet, daß es als Verfahren zur Herstellung von Acrolein durchgeführt wird.

37. Verfahren nach Anspruch 1, **dadurch gekennzeichnet, daß** es als Verfahren zur Herstellung von Acrylsäure durchgeführt wird.

## Claims

1. A process for the preparation of acrolein or acrylic acid or a mixture thereof from propane, in which
A) in a first stage A, the propane is subjected to a partial dehydrogenation under heterogeneous catalysis in the gas phase with formation of a product gas mixture A which contains molecular hydrogen, propylene and unconverted propane,
B) of the components contained in the product gas mixture A and differing from propane and propylene, at least a portion of the molecular hydrogen is separated off from said mixture A of stage A, containing molecular hydrogen, propylene and unconverted propane, the mixture is then used as product gas mixture A' in a second stage B for feeding at least one oxidation reactor and, in the at least one oxidation reactor, the propylene is subjected to a selective partial gas-phase oxidation with molecular oxygen under heterogeneous catalysis to give a product gas mixture B which contains acrolein and acrylic acid or a mixture thereof as the desired product, and
C) in a third stage C, the desired product is separated off from the product gas mixture B obtained in the partial oxidation of the propylene in stage B and at least unconverted propane contained in the product gas mixture of stage B is recycled to the dehydrogenation stage A,
wherein molecular nitrogen is present as diluent gas in the partial oxidation of the propylene in stage B.

2. A process as claimed in claim 1, wherein the feed gas mixture of the at least one oxidation reactor in the second stage B contains at least 5 mol%, based on propylene contained therein, of molecular nitrogen.

3. A process as claimed in claim 1, wherein the feed gas mixture of the at least one oxidation reactor in the second stage B contains at least 50 mol%, based on propylene contained therein, of molecular nitrogen.

4. A process as claimed in claim 1, wherein the feed gas mixture of the at least one oxidation reactor in the second stage B contains at least 100 mol%, based on propylene contained therein, of molecular nitrogen.

5. A process as claimed in any of claims 1 to 4, wherein the molar ratio of the amount of molecular nitrogen contained in the feed gas mixture of the at least one oxidation reactor in the second stage B to the amount of propane contained in the same feed gas mixture is at least 0.05.

6. A process as claimed in any of claims 1 to 4, wherein the molar ratio of the amount of molecular nitrogen contained in the feed gas mixture of the at least one oxidation reactor in the second stage B to the amount of propane contained in the same feed gas mixture is at least from 0.05 to 5.

7. A process as claimed in any of claims 1 to 4, wherein the molar ratio of the amount of molecular nitrogen contained in the feed gas mixture of the at least one oxidation reactor in the second stage B to the amount of propane contained in the same feed gas mixture is at least from 0.5 to 3.

8. A process as claimed in claim 1, wherein the composition of the feed gas mixture of the at least one oxidation reactor in the second stage B fulfills the following molar ratios:
propane : propene : N₂ : O₂ : H₂O : others
= from 0.5 to 20 : 1 : from 0.1 to 40 : from 0.1 to 10 : from 0 to 20 : from 0 to 1.

9. A process as claimed in claim 1, wherein the composition of the feed gas mixture of the at least one oxidation reactor in the second stage B fulfills the following molar ratios:
propane : propene : N₂ : O₂ : H₂O : others
= from 2 to 10 : 1 : from 0.5 to 20 : from 0.5 to 5 : from 0.01 to 10 : from 0 to 1.

10. A process as claimed in claim 1, wherein the composition of the feed gas mixture of the at least one oxidation reactor in the second stage B fulfills the following molar ratios:
propane : propene : N₂ : O₂ : H₂O : others
= from 3 to 6 : 1 : from 1 to 10 : from 1 to 3 : from 0.1 to 2 : from 0 to 0.5.

11. A process as claimed in any of claims 1 to 10, wherein the molar ratio of propylene contained in the product gas mixture A to molecular hydrogen contained in the product gas mixture is ≤ 100.

12. A process as claimed in any of claims 1 to 10, wherein the molar ratio of propylene contained in the product gas mixture A to molecular hydrogen contained in the product gas mixture A is ≤ 50.

13. A process as claimed in any of claims 1 to 10, wherein the molar ratio of propylene contained in the product gas mixture A to molecular hydrogen contained in the product gas mixture A is ≤ 10.

14. A process as claimed in any of claims 1 to 13, wherein the molar ratio of propylene contained in the product gas mixture A to molecular hydrogen contained in the product gas mixture A is ≥ 0.05.

15. A process as claimed in any of claims 1 to 14, wherein the propane conversion achieved in stage A is from 5 to 25 mol%, based on a single pass.

16. A process as claimed in any of claims 1 to 14, wherein the propane conversion achieved in stage A is from 10 to 20 mol%, based on a single pass.

17. A process as claimed in claim 15 or 16, wherein the propane to be dehydrogenated is diluted in stage A with steam.

18. A process as claimed in claim 17, wherein the molar ratio of steam to propane to be dehydrogenated is from 0.1 to 2.

19. A process as claimed in claim 15 or 16, wherein molecular hydrogen is added to the propane to be dehydrogenated.

20. A process as claimed in claim 19, wherein the molar ratio of molecular hydrogen to propane to be dehydrogenated is > 0 and ≤ 5.

21. A process as claimed in any of claims 15 to 20, wherein the partial dehydrogenation of the propane under heterogeneous catalysis in stage A is carried out adiabatically.

22. A process as carried out in any of claims 15 to 21, wherein the partial dehydrogenation of the propane under heterogeneous catalysis in stage A is carried out in a fixed-bed reactor through which the flow is axial or radial.

23. A process as claimed in any of claims 15 to 21, wherein the partial dehydrogenation of the propane under heterogeneous catalysis in stage A is carried out in a tray reactor.

24. A process as claimed in claim 23, wherein the temperature of the reaction gas mixture throughout the tray reactor is 450 to 550°C.

25. A process as claimed in claim 23 or 24, wherein the tray reactor contains from 2 to 8 catalyst beds spatially in succession.

26. A process as claimed in any of claims 23 to 25, wherein molecular oxygen is added to the reaction gas mixture during the partial dehydrogenation of the propane under heterogeneous catalysis in stage A.

27. A process as claimed in any of claims 1 to 26, wherein the amount of molecular hydrogen present is separated off from product gas mixture A before it is further used as product gas mixture A'.

28. A process as claimed in any of claims 1 to 26, wherein the components other than propane and propylene are separated off from product gas mixture A before it is further used as product gas mixture A'.

29. A process as claimed in any of claims 1 to 26, wherein the components other than propane and propylene are separated off from the product gas mixture A by bringing the product gas mixture A into contact with an organic solvent, adsorbing the propane and propylene selectively therein, liberating them again by subsequent desorption and using them as product gas mixture A' for feeding the at least one oxidation reactor in stage B.

30. A process as claimed in any of claims 1 to 29, wherein air is concomitantly used for feeding the at least one oxidation reactor of stage B.

31. A process as claimed in any of claims 1 to 30, wherein acrolein and/or acrylic acid contained in the product gas mixture B are separated off from said mixture, and at least a portion of the remaining residual gas is recycled to the dehydrogenation stage A.

32. A process as claimed in claim 28, wherein acrolein and/or acrylic acid contained in the product gas mixture B are separated off from said mixture, and the total amount of the remaining residual gas is recycled to the dehydrogenation stage A.

33. A process as claimed in any of claims 1 to 30, wherein acrolein and/or acrylic acid contained in the product gas mixture B are first separated off from said mixture, the components other than propane and propylene are separated off from the remaining residual gas, and the remaining propane and propene are recycled to the dehydrogenation stage A.

34. A process as claimed in any of claims 1 to 33, wherein stage B consists of two tube-bundle reactors connected in series.

35. A process as claimed in claim 23, wherein the tray reactor comprises at least one catalyst bed which catalyzes the combustion of hydrogen.

36. A process as claimed in claim 1, which is carried out as a process for the preparation of acrolein.

37. A process as claimed in claim 1, which is carried out as a process for the preparation of acrylic acid.

## Revendications

1. Procédé de préparation d'acroléine ou d'acide acrylique ou de leur mélange à partir de propane, procédé dans lequel
A) dans une première étape A, on soumet le propane à une déshydrogénation catalysée de manière hétérogène, partielle, dans la phase gazeuse avec formation d'un mélange gazeux produit A, qui contient de l'hydrogène moléculaire, du propylène et du propane n'ayant pas réagi,
B) dans le mélange gazeux produit A de l'étape A, qui contient de l'hydrogène moléculaire, du propylène et du propane n'ayant pas réagi, on isole, des éléments contenus dedans qui sont différents du propane et du propylène, au moins une quantité partielle de l'hydrogène moléculaire et on l'utilise ensuite, comme mélange gazeux produit A' dans une deuxième étape B pour charger au moins un réacteur d'oxydation et, dans ledit au moins un réacteur d'oxydation, on soumet le propylène à une oxydation partielle en phase gazeuse catalysée de manière hétérogène, sélective, par de l'oxygène moléculaire pour former un mélange gazeux produit B, qui contient de l'acroléine ou de l'acide acrylique ou leur mélange comme produit visé, et
C) à partir du mélange gazeux produit B obtenu dans l'étape B dans le cadre de l'oxydation partielle du propylène, on isole dans une troisième étape C le produit visé et on recycle dans l'étape de déshydrogénation A au moins le propane qui n'a pas réagi et qui est contenu dans le mélange gazeux produit de l'étape B,
**caractérisé en ce que**, dans le cadre de l'oxydation partielle du propylène dans l'étape B, on utilise conjointement de l'azote moléculaire, comme gaz de dilution.

2. Procédé suivant la revendication 1, **caractérisé en ce que** le mélange gazeux du chargement dudit au moins un réacteur d'oxydation de la deuxième étape B contient, par rapport au propylène contenu dedans, au moins 5 moles % d'azote moléculaire.

3. Procédé suivant la revendication 1, **caractérisé en ce que** le mélange gazeux du chargement dudit au moins un réacteur d'oxydation de la deuxième étape B contient, par rapport au propylène contenu dedans, au moins 50 moles % d'azote moléculaire.

4. Procédé suivant la revendication 1, **caractérisé en ce que** le mélange gazeux du chargement dudit au moins un réacteur d'oxydation de la deuxième étape B contient, par rapport au propylène contenu dedans, au moins 100 moles % d'azote moléculaire.

5. Procédé suivant l'une des revendications 1 à 4, **caractérisé en ce que** le rapport molaire entre la quantité d'azote moléculaire contenue dans le mélange gazeux du chargement dudit au moins un réacteur d'oxydation de la deuxième étape B et la quantité de propane contenue dans le même mélange gazeux du chargement est d'au moins 0,05.

6. Procédé suivant l'une des revendications 1 à 4, **caractérisé en ce que** le rapport molaire entre la quantité d'azote moléculaire contenue dans le mélange gazeux du chargement dudit au moins un réacteur d'oxydation de la deuxième étape B et la quantité de propane contenue dans le même mélange gazeux du chargement est d'au moins 0,05 à 5.

7. Procédé suivant l'une des revendications 1 à 4, **caractérisé en ce que** le rapport molaire entre la quantité d'azote moléculaire contenue dans le mélange gazeux du chargement dudit au moins un réacteur d'oxydation de la deuxième étape B et la quantité de propane contenue dans le même mélange gazeux du chargement est d'au moins 0,5 à 3.

8. Procédé suivant la revendication 1, **caractérisé en ce que** la composition du mélange gazeux du chargement dudit au moins un réacteur d'oxydation de la deuxième étape B répond aux proportions molaires suivantes :
propane/propène/N₂/O₂/H₂O/autres = 0,5 à 20/1/0,1 à 40/0,1 à 10/0 à 20/0 à 1.

9. Procédé suivant la revendication 1, **caractérisé en ce que** la composition du mélange gazeux du chargement dudit au moins un réacteur d'oxydation de la deuxième étape B répond aux proportions molaires suivantes :
propane/propène/N₂/O₂/H₂O/autres = 2 à 10/1/0,5 à 20/0,5 à 5/0,01 à 10/0 à 1.

10. Procédé suivant la revendication 1, **caractérisé en ce que** la composition du mélange gazeux du chargement dudit au moins un réacteur d'oxydation de la deuxième étape B répond aux proportions molaires suivantes :
propane/propène/N₂/O₂/H₂O/autres = 3 à 6/1/1 à 10/1 à 3/0,1 à 2/0 à 0,5.

11. Procédé suivant l'une des revendications 1 à 10, **caractérisé en ce que** le rapport molaire entre le propylène contenu dans le mélange gazeux produit A et l'hydrogène moléculaire contenu dans le mélange gazeux produit A est ≤ 100.

12. Procédé suivant l'une des revendications 1 à 10, **caractérisé en ce que** le rapport molaire entre le propylène contenu dans le mélange gazeux produit A et l'hydrogène moléculaire contenu dans le mélange gazeux produit A est ≤ 50.

13. Procédé suivant l'une des revendications 1 à 10, **caractérisé en ce que** le rapport molaire entre le propylène contenu dans le mélange gazeux produit A et l'hydrogène moléculaire contenu dans le mélange gazeux produit A est ≤ 10.

14. Procédé suivant l'une des revendications 1 à 13, **caractérisé en ce que** le rapport molaire entre le propylène contenu dans le mélange gazeux produit A et l'hydrogène moléculaire contenu dans le mélange gazeux produit A est ≥ 0,05.

15. Procédé suivant l'une des revendications 1 à 14, **caractérisé en ce que** la conversion de propane obtenue dans l'étape A est de 5 à 25 moles %, pour ce qui concerne un passage unique.

16. Procédé suivant l'une des revendications 1 à 14, **caractérisé en ce que** la conversion de propane obtenue dans l'étape A est de 10 à 20 moles %, pour ce qui concerne un passage unique.

17. Procédé suivant l'une des revendications 15 et 16, **caractérisé en ce que** le propane à déshydrogéner dans l'étape A est dilué par de la vapeur d'eau.

18. Procédé suivant la revendication 17, **caractérisé en ce que** le rapport molaire entre la vapeur d'eau et le propane à déshydrogéner est de 0,1 à 2.

19. Procédé suivant l'une des revendications 15 et 16, **caractérisé en ce qu'**au propane à déshydrogéner, on ajoute de l'hydrogène moléculaire.

20. Procédé suivant la revendication 19, **caractérisé en ce que** le rapport molaire entre l'hydrogène moléculaire et le propane à déshydrogéner est > 0 et ≤ 5.

21. Procédé suivant l'une des revendications 15 à 20, **caractérisé en ce que** la déshydrogénation catalysée de manière hétérogène, partielle, du propane dans l'étape A est effectuée de manière adiabatique.

22. Procédé suivant l'une des revendications 15 à 21, **caractérisé en ce que** la déshydrogénation catalysée de manière hétérogène, partielle, du propane dans l'étape A est effectuée dans un réacteur à lit fixe traversé axialement ou radialement.

23. Procédé suivant l'une des revendications 15 à 21, **caractérisé en ce que** la déshydrogénation catalysée de manière hétérogène, partielle, du propane dans l'étape A est effectuée dans un réacteur à claies.

24. Procédé suivant la revendication 23, **caractérisé en ce que** la température du mélange gazeux réactionnel est de 450 à 550°C dans la totalité du réacteur à claies.

25. Procédé suivant l'une des revendications 23 et 24, **caractérisé en ce que** le réacteur à claies contient spatialement successivement 2 à 8 lits de catalyseur.

26. Procédé suivant l'une des revendications 23 à 25, **caractérisé en ce qu'**on ajoute de l'oxygène moléculaire au mélange réactionnel pendant la déshydrogénation catalysée de manière hétérogène, partielle, du propane dans l'étape A.

27. Procédé suivant l'une des revendications 1 à 26, **caractérisé en ce qu'**on isole la quantité contenue d'hydrogène moléculaire à partir du mélange gazeux produit A avant son utilisation ultérieure comme mélange gazeux produit A'.

28. Procédé suivant l'une des revendications 1 à 26, **caractérisé en ce qu'**à partir du mélange gazeux produit A, on isole, avant son utilisation ultérieure comme mélange gazeux produit A', les éléments différents du propane et du propylène.

29. Procédé suivant l'une des revendications 1 à 26, **caractérisé en ce que**, à partir du mélange gazeux produit A, on isole les éléments différents du propane et du propylène par le fait qu'on met en contact le mélange gazeux produit A avec un solvant organique, on absorbe dans celui-ci le propane et le propylène de manière sélective, on les libère à nouveau par une désorption ultérieure et on les utilise, comme mélange gazeux produit A', pour le chargement dudit au moins un réacteur d'oxydation de l'étape B.

30. Procédé suivant l'une des revendications 1 à 29, **caractérisé en ce qu'**on utilise conjointement de l'air pour le chargement dudit au moins un réacteur d'oxydation de l'étape B.

31. Procédé suivant l'une des revendications 1 à 30, **caractérisé en ce qu'**à partir du mélange gazeux produit B, on isole l'acroléine et/ou l'acide acrylique contenus dedans et **en ce que**, du gaz résiduaire subsistant, on recycle au moins une quantité partielle dans l'étape de déshydrogénation A.

32. Procédé suivant la revendication 28, **caractérisé en ce qu'**à partir du mélange gazeux produit B, on isole l'acroléine et/ou l'acide acrylique contenus dedans et **en ce qu'**on recycle la quantité totale du gaz résiduaire subsistant dans l'étape de déshydrogénation A.

33. Procédé suivant l'une des revendications 1 à 30, **caractérisé en ce qu'**à partir du mélange gazeux produit B, on isole tout d'abord l'acroléine et/ou l'acide acrylique contenus dedans, on isole à partir du gaz résiduaire subsistant les éléments différents du propane et du propylène et on recycle le propane et le propène subsistants dans l'étape de déshydrogénation A.

34. Procédé suivant l'une des revendications 1 à 33, **caractérisé en ce que** l'étape B est constituée de deux réacteurs à faisceaux tubulaires montés l'un derrière l'autre.

35. Procédé suivant la revendication 23, **caractérisé en ce que** le réacteur à claies comporte au moins un lit de catalyseur qui catalyse la combustion de l'hydrogène.

36. Procédé suivant la revendication 1, **caractérisé en ce qu'**il est effectué sous la forme d'un procédé de préparation d'acroléine.

37. Procédé suivant la revendication 1, **caractérisé en ce qu'**il est effectué sous la forme d'un procédé de préparation d'acide acrylique.
